(19) Europäisches Patentamt
European Patent Office
Office européen des brevets

(11) EP 2 908 837 B1

(12) **EUROPEAN PATENT SPECIFICATION**

(45) Date of publication and mention of the grant of the patent:
**13.02.2019 Bulletin 2019/07**

(21) Application number: **13783290.3**

(22) Date of filing: **18.10.2013**

(51) Int Cl.:
***C07K 7/06*** *(2006.01)*

(86) International application number:
**PCT/EP2013/071898**

(87) International publication number:
**WO 2014/060601 (24.04.2014 Gazette 2014/17)**

(54) **COMPOSITIONS FOR DRUG DELIVERY**

ZUSAMMENSETZUNGEN ZUR WIRKSTOFFFREISETZUNG

COMPOSITIONS POUR L'ADMINISTRATION DE MÉDICAMENTS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.10.2012 US 201213655954**

(43) Date of publication of application:
**26.08.2015 Bulletin 2015/35**

(73) Proprietors:
- **Vect-Horus**
  **13344 Marseille Cedex 15 (FR)**
- **Centre National de la Recherche Scientifique**
  **75016 Paris (FR)**
- **Universite d'Aix Marseille**
  **13284 Marseille Cedex 07 (FR)**

(72) Inventors:
- **JACQUOT, Guillaume**
  **13100 Aix en Provence (FR)**
- **LECORCHE, Pascaline**
  **13004 Marseille (FR)**
- **MALCOR, JD**
  **Cambridge CB1 1LA (GB)**
- **PAYROT, Nadine**
  **66000 Perpignan (FR)**
- **DAVID, Marion**
  **13010 Marseille (FR)**
- **MOLINO, Yves**
  **13820 Ensues la Redonne (FR)**
- **KHRESTCHATISKY, Michel**
  **13007 Marseille (FR)**

(74) Representative: **Cabinet Becker et Associés**
**25, rue Louis le Grand**
**75002 Paris (FR)**

(56) References cited:
**WO-A2-2011/131896**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 908 837 B1

SEQUENCE LISTING

<110> VECT-HORUS CNRS UNIVERSITE AIX MARSEILLE

<120> COMPOSITIONS AND METHODS FOR DRUG DELIVERY

<130> B1687PC00

<160> 5

<170> PatentIn version 3.3

<210> 1
<211> 8
<212> PRT
<213> Artificial

<220>
<223> peptide

<220>
<221> MISC_FEATURE
<222> (1)..(1)
<223> Xaa = (D)-cys

<220>
<221> MISC_FEATURE
<222> (3)..(3)
<223> Xaa = Thz

<220>
<221> MISC_FEATURE
<222> (8)..(8)
<223> Xaa = Pen

<400> 1

```
Xaa Met Xaa Arg Leu Arg Gly Xaa
1               5
```

<210> 2
<211> 8
<212> PRT
<213> Artificial

<220>
<223> peptide

<220>
<221> MISC_FEATURE
<222> (1)..(1)
<223> Xaa = (D)-Cys

<220>
<221> MISC_FEATURE
<222> (3)..(3)
<223> Xaa = Thz

<220>
<221> MISC_FEATURE
<222> (7)..(7)
<223> Xaa = Sar

<220>
<221> MISC_FEATURE
<222> (8)..(8)
<223> Xaa = Pen

<400> 2

```
Xaa Met Xaa Arg Leu Arg Xaa Xaa
1               5
```

<210> 3
<211> 8
<212> PRT
<213> Artificial

<220>
<223> peptide

<220>
<221> MISC_FEATURE
<222> (1)..(1)
<223> Xaa = D-Cys

<220>
<221> MISC_FEATURE
<222> (3)..(3)
<223> Xaa = Pip

<220>
<221> MISC_FEATURE
<222> (7)..(7)
<223> Xaa = Sar

<400> 3

```
Xaa Met Xaa Arg Leu Arg Xaa Cys
1               5
```

<210> 4
<211> 8
<212> PRT
<213> Artificial

<220>
<223> peptide

<220>
<221> MISC_FEATURE
<222> (1)..(1)
<223> Xaa = (D)-cys

<220>
<221> MISC_FEATURE
<222> (3)..(3)
<223> Xaa = Pip

<220>
<221> MISC_FEATURE
<222> (8)..(8)
<223> Xaa = Pen

<400> 4

```
Xaa Met Xaa Arg Leu Arg Gly Xaa
1               5
```

<210> 5
<211> 8
<212> PRT
<213> Artificial

<220>
<223> Peptide

<220>
<221> MISC_FEATURE
<222> (1)..(1)
<223> Xaa = (D)-cys

<220>
<221> MISC_FEATURE
<222> (3)..(3)
<223> Xaa = Pip

<220>
<221> MISC_FEATURE
<222> (7)..(7)
<223> Xaa = Sar

<220>
<221> MISC_FEATURE
<222> (8)..(8)
<223> Xaa = Pen

<400> 5

```
Xaa Met Xaa Arg Leu Arg Xaa Xaa
1               5
```

## Description

The invention relates to compositions and methods suitable for delivering molecules to a mammal. The invention particularly relates to peptide derivatives (peptides and pseudo-peptides), dimers or multimers thereof, and to their use as vectors for molecules of interest. The invention also relates to conjugates containing a peptide derivative of the invention or a dimer or multimer thereof bound to a molecule of interest. The peptides of the invention can be used, in particular, to vectorize via specific receptors (receptor-mediated transport, RMT), generally in the form of prodrug conjugates, molecules of pharmaceutical or diagnostic interest such as, for example, therapeutic molecules, imaging or diagnostic agents, or molecular probes, across cell membranes of different tissues or organs (such as liver, adrenal gland and intestine), healthy or pathologic (cancer cells), and in particular to enable their transport across physiological barriers of the nervous system such as the Blood brain barrier (BBB), Blood-spinal cord barrier (BSCB), or Blood-retinal barrier (BRB).

CONTEXT OF THE INVENTION

According to *IMS Health,* the global market for drugs for treating central nervous system (CNS, brain and spinal cord) pathologies was approximately 70 billion dollars in 2007, with nearly 9 billion dollars of this amount representing products arising from drug delivery technologies (Jain, 2008, Jain PharmaBiotech Report, Drug Delivery in CNS disorders). Thus, neurology is today one of the three largest therapeutic areas, along with cardiovascular medicine and oncology. Although the number of people suffering from CNS disorders and pathologies throughout the world is larger than that of people with cardiovascular diseases or cancers, neurology remains an under-developed market. This is explained in part by the fact that 98% of potential drugs for treating CNS pathologies do not cross the blood-brain barrier or BBB (Pardridge, 2003, Mol. Interv., 3, 90-105). Only 5% of the drugs presently on the market relate to the nervous system.

Indeed, the CNS is protected from potentially toxic substances by the presence of two principal physiological barrier systems: the BBB/BSCB, and the blood-cerebrospinal fluid barrier (BCSFB). The BBB is regarded as the principal route for the uptake of plasma ligands at the brain level. Its surface area is approximately 5000 times larger than that of the BCSFB. The overall length of the constitutive blood vessels of the BBB is approximately 600 km. Each $cm^3$ of cerebral cortex contains the equivalent of 1 km of blood vessels. The total surface area of the BBB is estimated at 20 $m^2$ (De Boer et al., 2007, Clin. Pharmacokinet., 46(7), 553-576). The BBB and BSCB are regarded as major obstacles to overcome in the development of novel therapies for treating CNS pathologies and injuries (Neuwelt et al., 2008, Lancet Neurol., 7, 84-96).

Similarly, in the eye, the blood-retinal barrier (BRB), is part of the blood-ocular barrier that consists of cells that are also joined tightly together and that prevent certain substances from entering the retina. The BRB has two components : the retinal vascular endothelium and the retinal pigment epithelium also referred to as inner and outer components (inner BRB [iBRB] and outer BRB [oBRB]). Retinal blood vessels that are similar to cerebral blood vessels maintain the inner blood-ocular barrier. Like the BBB or BSCB this physiological barrier comprises a single layer of non-fenestrated endothelial cells, with tight junctions (TJ). These junctions between retinal epithelial cells prevent passage of large molecules from choriocapillaris into the retina.

In 2007, the total worldwide ophthalmic pharmaceutical sector was valued at approximately $11.52 billion (Visiongain, Ophthalmics. 2007, Visiongain, Inc.: San Francisco. Janoria KG, et al. Novel approaches to retinal drug delivery. Expert Opin Drug Deliv. 2007;4(4):371-388). Retinal diseases include retinitis pigmentosa, macular degeneration, cone-rod dystrophy (CORD), retinal separation, retinal detachment, hypertensive retinopathy and diabetic retinopathy, retinoblastoma, lipemia retinalis etc.

Due to the increasing age of many populations, financial experts forecast average yearly expansion in the sector to be greater than 10% over the near term. Nonetheless, the sustained delivery of therapeutically effective concentrations of drug to treat diseases of the eye, particularly diseases of the posterior segment of the eye, remains a significant technological challenge. The corresponding problem of patient compliance with existing therapies is a critical issue as well. Thus, substantial market opportunities exist for companies that pursue novel molecules as well as innovative drug delivery systems.

Thus, BBB, BSCB, and BRB represent major obstacles to the use of potential drugs against many CNS and eye disorders, but also a large surface of potential exchange between the blood and nervous tissue.

As a general rule, only a few small lipophilic molecules of approximately 450 to 600 Daltons (only 2% of drug candidates) can pass through the above-mentioned physiological barriers, that is to say, pass from the blood to the nervous tissue. The molecular weight and the size of many drug candidates, which show promising results in *in vitro* studies and in animal studies for treating CNS disorders, are considerably larger. Thus, most molecules such as therapeutic peptides, proteins, including therapeutic antibodies are generally excluded from passage/transport from the blood to nervous tissue in the CNS or the eye (retina), because of the low transcellular permeability of nervous tissue capillary

endothelial cells. Brain capillary endothelial cells (BCECs) organized in vessels are surrounded by a basal lamina, astrocyte end-feet, pericytes and microglial and neuronal cells. The tight association of endothelial cells with astrocyte end-feet is responsible for the development and maintenance of properties of BBB impermeability to most molecules, thus ensuring strict and effective control of molecular exchanges between the blood and the brain in order to maintain brain homeostasis. Endothelial cells are closely bound by TJ, compared with endothelial cells of other organs, which are fenestrated. These TJ thus prevent paracellular passage across the BBB. Endothelial cells and the astrocyte end-feet, which surround them, also constitute a physiological barrier, since these cells have effective efflux systems, which restrict any passage/transport by the transcellular route. Indeed, certain molecules capable of crossing physiological barriers are actively expelled from the endothelial cells towards the blood system by multidrug resistant (MDR) transport proteins. These active efflux transport (AET) systems generally control the active efflux of small molecules from the nervous tissue towards the blood system. As an example, the model AET system at the BBB is the ATP binding cassette (ABC) transporter, namely P-glycoprotein (P-gp); however, other AET systems are present at the BBB such as MDR-associated protein 1 (MRP1). P-gp, which is principally located on the luminal surface of BCECs is an essential element in the function of the physiological barrier of the BBB preventing entry into the brain of most xenobiotics but also drug candidates and other molecules of therapeutic interest capable of being active in the CNS. These properties thus strongly limit the passage of substances from the blood plasma towards the extracellular space of nervous tissues in the CNS and the eye.

One of the reasons that may explain why no really effective treatment is currently available for the principal CNS or ocular pathologies and injuries (brain cancer, Parkinson's and Alzheimer's diseases, cerebrovascular accidents (CVA), etc.) is that developers of drug candidates for treating brain pathologies carry out in-house research programs (brain drug-discovery programs) while investing little effort in the problems of passing the barriers and in the preferential targeting of nervous tissues, and notably of the brain (brain drug-targeting programs), (Pardridge, 2003, Mol. Interv., 3, 90-105). A drug candidate must follow certain structural, physicochemical, pharmacochemical and pharmacological rules in order to have the best chances of becoming a drug for treating a CNS pathology or disorder (Pajouhesh et al., 2005, NeuroRx, 2(4), 541-553). Thus, in the development of a drug candidate, the selectivity and specificity (pharmacological profiling) of a molecule for its target are essential to its therapeutic activity (effectiveness). The bioavailability and potential toxicity (pharmaceutical profiling) of a molecule are crucial for its future as a drug. In other words, any molecule likely to become a drug for treating a CNS or ocular pathology or disorder must move across the BBB/BSCB or the BRB, maintain its biological activity, and exhibit suitable properties of pharmacokinetics (PK), absorption, distribution, metabolism and excretion/elimination (ADME) and pharmacodynamics (PD), with low toxicity (Tox). In fact, the hydrophilic/lipophilic balance of the molecule under development is particularly difficult to find for medicinal chemists in this field of nervous system therapeutics.

One of the major problems in treating CNS and eye disorders and pathologies thus lies in the fact that the molecules administered do not pass the BBB/BSCB/BRB and thus cannot reach their target(s) in the CNS or the eye. One of the priorities of research in the discovery of molecules for treating, diagnosing or imaging CNS or ocular disorders or pathologies is thus to find means for increasing the effectiveness of passage of active substances across the BBB/BSCB or BRB.

In this respect, strategies for the vectorization of molecules across these barriers, currently studied and used by developers of drug candidates in order to enable a molecule of therapeutic interest to reach the CNS, can be divided into two principal strategies: pharmacological approaches and physiological approaches (Pardridge, 2007, Pharm. Res., 24(9), 1733-1744; De Boer et al., 2007, Clin. Pharmacokinet., 46(7), 553-576; De Boer et al., 2007, Annu. Rev. Pharmacol. Toxicol., 47, 327-355; Jones et al., 2007, Pharm. Res., 24(9), 1759-1771*;* Vlieghe and Khrestchatisky, 2012, Med Res Rev., 33(3), 457-516).

*Invasive approaches*

Invasive approaches can be implemented by direct intraventricular injection in the brain, intracerebral injection or intrathecal infusion of the active substance, or by disruption of the BBB/BSCB (temporary rupture of the integrity of these barriers).

The principal problem of neurosurgical approaches by intraventricular injection, apart from the costs relating to the neurosurgical procedure, is that the drug is not delivered directly in the brain parenchyma but in the cerebrospinal fluid. Intraventricular infusion involves placing a catheter in the ventricles (Aird, 1984, Exp. Neurol., 86, 342-358). This highly invasive technique is not effective for the transport of active substances in the nervous parenchyma. Indeed, in the brain for example, the flow volume from the cerebrospinal fluid to the brain parenchyma during delivery of a drug by intraventricular infusion is governed by an abnormally slow diffusion of its convection (transport), because the brain does not have intraparenchymal volumetric flow.

Similarly for intracerebral injection, the diffusion of an active substance in the brain decreases very quickly from the injection site to the site of the lesion. Indeed, the cerebral concentration of an active substance decreases by 90%

at a distance of 500 μm from its injection site.

Intrathecal infusion involves placing a catheter in the brain. This catheter is connected to a pump that delivers the active substance at a predefined flow rate. Owing to the fact that the brain is the only organ that does not have a lymphatic system, normally serving to transport extracellular fluids back to general circulation, the distribution of an active substance by intrathecal infusion in the brain is very slow. This decreases the concentration of the active substance at the site of the lesion.

Moreover, risks of infection are significant during such neurosurgical procedures, notably by the presence of the catheter. Under these conditions, patient comfort is not optimal.

The temporary opening of the BBB is associated with transitory opening of the TJ of BCECs. This is the case for vasoactive substances such as leukotrienes or bradykinins (Baba et al., 1991, J. Cereb. Blood Flow Metab., 11, 638-643). This strategy is equally invasive and requires arterial access to the carotid in sedated subjects/patients. The major problem encountered by the temporary rupture of the integrity of the BBB, besides expenses relating to the radiological procedure for access to the carotid, is that the BBB only remains open for a short period of time, thus limiting the possibility of delivering a drug over an extended period. Moreover, the temporary rupture of the BBB allows plasma proteins to enter the brain (whereas these proteins can be toxic for the brain) and can also facilitate the entry of infectious agents. This type of rupture of the BBB can thus lead to chronic neuropathologic disruptions and is associated with high risks of infection (Salahuddin et al., 1988, Acta Neuropathol., 76, 1-10).

*Pharmacological approaches to vectorization*

Pharmacological strategies for transporting molecules include transcellular diffusion of molecules made more hydrophobic by the addition of lipid or lipophilic groups on the active substance (transcellular lipophilic diffusion, TLD) or the use of liposomes (Zhou et al., 1992, J. Control. Release, 19, 459-486), and transport by ionic adsorption via positively charged vector molecules or by cationization of the active molecule (adsorptive-mediated transport or AMT).

The addition of a lipid or lipophilic group enables the chemical conversion of hydrophilic molecules into more hydrophobic molecules notably through prodrug approaches. However, the synthesis of such compounds leads to molecules that exceed the optimal transport threshold to cross the BBB/BSCB/BRB, notably with regard to molecular weight (MW), which becomes greater than the optimal limit of 450 Daltons (Pajouhesh et al., 2005, NeuroRx, 2(4), 541-553). For the same reason, liposomes or even small vesicles (micelles, etc.) or nanoparticles (nanospheres, nanocapsules) are generally too large, are not specific enough for the BBB/BSCB/BRB, and consequently are relatively less effective for transporting molecules of therapeutic interest (or imaging or diagnostic agents, or any other molecule such as a molecular probe) across these barriers (Levin, 1980, J. Med. Chem., 23, 682-684; Schackert et al., 1989, Selective Cancer Ther., 5, 73-79). Moreover, this type of vesicle system generally has non-negligible toxic effects in the cerebrum. Thus, the principal problems encountered by lipidization technologies are their low specificity for specifically targeting and crossing the BBB/BSCB/BRB compared to other cell membranes, the decrease in the plasma values of the area under the curve (AUC) of the drug, and their generally limited use for vectorization of small molecules.

In AMT (addition of a cationic group via covalent bonding or direct cationization of the drug), the principal problem encountered is the low specificity for targeting and crossing specifically the BBB/BSCB/BRB compared to other cell membranes. Indeed, AMT is based on cationic molecules adsorbing on cells whose membrane is negatively charged, which is the case for most cells. The decrease in plasma values of the AUC of the drug, their generally limited use for vectorization of small molecules, and their cytotoxicity are additional factors which penalize the AMT vectorization approach.

*Physiological approaches to vectorization*

Strategies based on physiological approaches to vectorization consist in exploiting the various natural transport mechanisms of the barriers. These mechanisms of active transport of molecules across the BBB work either via coupling with a specific receptor substrate or by molecular mimicry with a specific receptor substrate (carrier-mediated transport or CMT), or via coupling or fusion with a ligand specifically targeting a receptor (RMT, receptor-mediated transport or receptor-mediated transcytosis).

As an example, molecules such as L-DOPA (Parkinson's disease), melphalan (brain cancer), α-methyl-DOPA (arterial hypertension) and gabapentin (epilepsy) pass into the brain by CMT via large neutral amino-acid transporters 1 and 2 (LAT1 and LAT2), (Pardridge, 2003, Mol. Interv., 3, 90-105). These molecules have chemical structures close to phenylalanine, one of the natural substrates of LAT1. However, the principal problems encountered by CMT approaches are their broad selectivity/specificity for conjugates that closely imitate/mimic the substrate of the endogenous receptor/transporter, and consequently their use which remains limited to vectorization of small molecules.

RMT calls upon a receptor-dependent transport system. Vectorization is carried out via mechanisms of endocytosis by targeting the endogenous receptors present in the target tissue including brain capillaries. Notable examples

of the various human BBB receptors which would be involved in RMT include: transferrin receptor (TfR) which transports transferrin (Tf) bound to iron, insulin receptor (IR) or insulin-like growth factor receptor (IGFR), receptors which enable the transport of cholesterol contained in low density, high density, and very low density lipoproteins (LDL, HDL et VLDL respectively), including low-density lipoprotein (LDL) receptor and members of the family of low-density lipoprotein receptor-related protein (LRP), diphtheria toxin receptor (DTR) or heparin binding epidermal growth factor-like growth factor (HB-EGF), as well as scavenger receptors (SCAV-Rs) including scavenger receptor class B type I (SR-BI). In RMT, the receptors on the membrane of specific cells in organs, including BBB endothelial cells bind their ligand, which leads to endocytosis of the complex composed of the receptor and its ligand in a vesicle, which forms on the cell surface and then penetrates the target cell. This RMT process does not appear to depend on the size of the molecule engulfed by endocytosis. In the case of the BBB/BSCB/BRB, the ligand/receptor complex or the dissociated ligand can pass through the endothelial cell (transcytosis), and can thus cross these physiological barriers to act in nervous tissue. Thus, RMT is a mechanism that enables transport into specific cell types or organs, and notably from the blood to the CNS or eye, of many molecules or molecular complexes. Receptors for these molecules or molecular complexes can be used to transport the natural ligands of these receptors modified to carry drugs, and/or nanoparticles. For example, coating of nanoparticles with polysorbates, especially polysorbate 80 leads to the adsorption of apolipoprotein E from blood plasma onto the nanoparticle surface which then mimic low density lipoprotein (LDL) particles and could interact with the LDLR leading to their uptake by the endothelial cells (Kreuter, 2012, Adv Drug Deliv Rev. pii: S0169-409X(12)00275-X). LDL receptor-targeted liposome-encapsulated doxorubicin increases the *in vitro* drug delivery across BBB cells (Pinzón-Daza et al., 2012, Br J Pharmacol. doi: 10.1111/j.1476-5381.2012.02103.x). Tf is the natural ligand of the TfR present on the BBB. Active substances have been coupled/conjugated to Tf for active transport across the BBB by the Tfr (Jefferies et al., 1984, Nature, 312, 162-163; Friden et al., 1983, Science, 259, 373-377*;* Friden, 1994, Neurosurgery, 35, 294-298). Although this vectorization strategy using a protein-type macromolecule enables an increase in the passage of the conjugate molecules of interest across the barriers, it has several disadvantages. First, the molecule is generally coupled/conjugated to the vector by gene expression methods (fusion) thus limiting the number of molecules to be transported to only polypeptides or proteins. Second, the system for coupling/conjugating the molecule with the vector is rather complex; traditional chemical or biochemical coupling does not yield well-defined macromolecular systems from a structural and molecular point of view. Moreover, potential competition between the conjugates and the endogenous ligands for the targeted receptor can lead either to an inhibition of the physiological process of RMT or to a decrease in the concentration of the endogenous ligands required for the proper functioning of the brain. Finally, RMT receptors can also be involved in cell signaling processes in the cerebrum and the conjugates could potentially interfere with these processes.

RMT via specific receptors can also be used to target drugs into other tissues/organs than the BBB and the brain. It has been shown for instance that uptake of cholesterol by the retina occurs primarily via a LDLR-mediated process (Tserentsoodol et al., 2006, Molecular Vision 2006; 12:1306-18).

The vascular system of organs, the parenchyma of specific organs, or diseased tissues can express high levels of a given receptor which can be used for drug targeting (*reviewed in* Chung and Wasan, 2004, Adv Drug Deliv Rev. 2004 May 7;56(9):1315-34). The LDLR for instance is expressed at high levels in the liver (in particular in the sinusoidal side of hepatocytes) and in other tissues such as the adrenal gland and intestine (Beisiegel et al., 1981, J Biol Chem. 25;256(8):4071-8 *;* Huettinger et al., 1984, J Clin Invest.; 74(3): 1017-26*;* Fong et al., 1989, J Clin Invest; 84(3): 847-56).

Finally, there is ample evidence for LDLR increased expression in numerous types of cancers and for LDL- or nanoparticule-mediated anticancer drug delivery via these LDLR of cancer cells including glioblastoma (Varshosaz et al., 2012, Eur J Med Chem; 54:429-38 *;* Kopecka et al., 2011 ; Journal of Controlled Release ; 149 :196-205 *;* Nikanjam et al., 2007, Int J Pharm.; 328(1): 86-94 *; reviewed in* Ng et al., 2011, Acc Chem Res;44(10):1105-13 *;* Firestone, 1994, Bioconjug Chem.;5(2):105-13.).

It has also been shown that several LDLR endogenous ligands such as LDLs or PCSK9, after binding to LDLR, undergo receptor-mediated-endocytosis followed by intracellular trafficking of membrane vesicles (endosomes) that ultimately fuse with lysosomes (Issandou et al., 2004, Biochem Pharmacol.; 67(12) :2281-9 *; reviewed in* Lambert et al., 2009, Atherosclerosis.; 203(1): 1-7).

Lysosomal Storage Diseases (LSD) represent about 70 genetically distinct conditions, with a combined birth frequency of about 1 in 7500. Enzyme replacement therapies (ERT) require uptake of the recombinant enzymes considered for treatment of the multiple LSD. The mannose-phosphate receptor (M6PR) is currently a major target for enzyme delivery to tissues and to the lysosomal compartment of cells (*Reviewed in* Cox, 2012, J Pathol.; 226(2) :241-54 *;* Lachmann, 2011, Curr Opin Pediatr. ; 23(6): 588-93), but other receptors, such as those involved in RMT could be considered, in particular for targeting ERT to the CNS, considering that approximately 2/3 of LSD affect the CNS.

International patent application WO2010/046588 describes for the first time peptides or pseudo-peptides that bind human, mice or rat LDLR and are able to carry across the BBB substances that can be of high MW and/or large volume.

The present application relates to novel peptides that bind LDLR, particularly human, mice and/or rat LDLR,

and which are optimized for addressing molecules towards LDLR-expressing tissues (such as liver, adrenal gland, intestine), healthy or pathologic (cancer cells), and in particular across the physiological barriers of the nervous system such as the BBB, BSCB or BRB.

## SUMMARY OF THE INVENTION

The present invention provides new peptides or pseudo-peptides capable of transporting molecules of interest across cell membranes of LDLR-expressing tissues, and in particular across physiological barriers of the CNS or retina (BBB/BSCB and BRB). The invention thus makes it possible to design new therapeutic or diagnostic agents with improved biodistribution or bioavailability, and notably with improved access (targeting) to the CNS and/or the eye and/or other organs enriched in LDLR such as liver, adrenal gland and intestine, and/or towards different cancer cells enriched in LDLR, and/or towards lysosomal compartments of cells.

In WO2010/046588 and WO2011/131896, the inventors have developed peptide derivatives capable of binding LDLR and of addressing molecules to the brain, including molecules of therapeutic or diagnostic interest.

The inventors have now designed and validated novel peptides exhibiting advantageous properties for the transport of molecules. The inventors have also designed novel multimers of such peptides which exhibit improved affinity and high transport capacity for the delivery of any agent to an animal. These novel peptides and multimers are capable of binding LDLR without competition with the natural ligand, and thus without interference with the transport of endogenous LDL, and represent novel products that are particularly advantageous for the delivery, targeting, or vectorization of therapeutic or diagnostic (including imaging) drugs or agents, notably to reach the CNS and/or the eye and/or other organs enriched in LDLR (e.g., liver, adrenal gland, intestine), and/or towards cancer cells enriched in LDLR, and/or towards lysosomal compartments of cells.

One object of the invention particularly relates to a peptide selected from SEQ ID NO: 1-3 and 5.

A further object of the invention is a multimeric agent of the following formula (A):

$$P_h\text{-}M\text{-}P_i \qquad (A)$$

wherein,

. each P represents the same or a different peptide of amino acid sequence A1-Met-A2-Arg-Leu-Arg-A3-A4 wherein A1 and A4 independently represent a cysteine or an analogue thereof selected from (D)-cysteine, penicillamine (Pen) and (D)-penicillamine ((D)-Pen), A2 represents a proline or an analogue thereof selected from pipecolic acid (Pip) and thiazolidine-4-carboxylic acid (Thz), and A3 represents a glycine or or sarcosine (Sar);
. M is a cross-linking group, and
. h and i are, independently from each other, an integer selected from 1, 2, 3, 4, or 5.

In a preferred embodiment, P is a peptide or pseudo-peptide of the following general formula (I'):

(D)-Cys-Met-A2-Arg-Leu-Arg-A3-A4 (I')

wherein A2 represents proline, Pip or Thz; A3 represents glycine (Gly) or sarcosine (Sar) and A4 represents cysteine, (D)-Cysteine, Penicillamine (Pen), and-or (D)-Penicillamine ((D)-Pen).

In a most preferred embodiment, P is a peptide selected from anyone of SEQ ID NOs: 1-5.

In a most preferred embodiment, h = i = 1.

The peptides and multimers of the invention advantageously have the ability to bind human LDL receptor (hLDLR), mice LDLR, or rat LDLR with high affinity, and to transport molecules of interest into LDLR-expressing tissues and cells, or across LDLR-expressing cells such as endothelial cells.

In this regard, another object of the invention is a peptide or multimer as defined above conjugated to one or several molecules, preferably to one or several diagnostic or therapeutic molecules.

The invention also relates to any conjugated compound of the following formula (III):

VxSzDy (III)

wherein V represents a peptide or multimer such as defined above, S represents a spacer, D represents an active substance or a substance of interest, x is an integer between 1 and 5 and y and z are integers between 1 and 10 .

Another object of the invention relates to a pharmaceutical composition comprising at least one conjugated

compound such as defined above, and one or more pharmaceutically acceptable excipients.

Another object of the invention relates to a diagnostic composition comprising a diagnostic or imaging agent conjugated to a peptide or pseudo-peptide or multimer such as defined above.

The invention also discloses a method for improving or enabling the passage of a molecule across cell membranes of LDLR-expressing tissues, healthy or pathologic, and in particular across the physiological barriers BBB/BSCB and BRB, comprising the coupling of said molecule to a peptide or pseudo-peptide or multimer such as defined above.

The invention also discloses an improved method for treating a pathology in a subject with a drug, the improvement consisting in coupling said drug to a peptide or pseudo-peptide or multimer such as defined above.

The invention can be used in any animal, particularly any mammal, and more particularly in human beings.

BRIEF DESCRIPTION OF FIGURES

Figure 1. Comparative diagram of synthesis in tandem and synthesis via a linker of a Peptide/molecule of therapeutic interest conjugate.

Figure 2. General scheme of synthesized peptides conjugated with reporter or fluorescent molecules such as Biotins, fluoresceins, (FITC), rhodamines (rhodamine RED-X), cyanines (Cy3.5) or Stag peptide, with a C-terminus (C-term) spacer/linker.

Figure 3. Pulse-chase experiment using CHO-hLDLR-GFP cells grown on coverslips and incubated with DiI-LDL 20 mg/mL together with the peptide-STag conjugate (SEQ ID NO: 1) 10 μM. After 30 minutes incubation at 4°C, cells are extensively washed and then incubated in chase media for 5 minutes or 3 hours to allow endocytosis of bound peptides and intracellular trafficking. Cell nuclei are stained using Hoechst. Insets show higher magnification of representative areas of confocal images and indicate colocalization of the peptide with both the LDLR and LDL during the early steps of the peptide and LDL trafficking (T5), and late accumulation of the peptide either in LDL-containing lysosomes that do not contain LDLR (T180 - Inset 2) or in LDLR-containing recycling vesicles that do not contain LDL (T180 - Inset 1).

Figure 4. The human Fc fragment of an IgG1 antibody was used as a prototype protein moiety to be transported into cells via the peptide vectors chemically linked to SEQ ID NO: 1. A fusion with reference peptide2 (termed "SEQ ID NO: 43", the amino acid sequence of which being disclosed as SEQ ID NO: 43 in WO2010/046588), prepared by recombinant molecular techniques, was used for comparison purpose. Pulse-chase experiment showing binding of the Fc-SEQ ID NO: 1 (chemically linked) and of the Fc-SEQ ID NO: 43 (fusion) to CHO-hLDLR-GFP cells.

CHO-hLDLR-GFP cells grown on coverslips were incubated for 30 minutes at 4°C with the chemically linked Fc-SEQ ID NO: 1, the fusion protein Fc-SEQ ID NO: 43 or Fc alone at 5 nM, extensively washed and then incubated in chase media for 5 minutes at 37 °C to allow endocytosis of bound proteins. Cell nuclei are stained using Hoechst. Fc is detected with an anti-human Fc antibody conjugated to Alexa 594. Note that only Fc-SEQ ID NO: 1 and Fc-SEQ ID NO: 43 bind hLDLR. Insets show higher magnification of representative areas of confocal images and indicate regions where LDLR and the Fc-peptide conjugates co-localize in early/sorting endosomes.

Figure 5. *In vitro* blood-brain barrier (BBB) model: rat brain capillary endothelial cells (RBCEC) co-cultured with astrocytes. In A, live RBCEC were incubated with DiI-LDL at 40μg/mL during 30 min incubation. In B, rat LDLR labelling at the surface of live RBCEC with a goat antibody that recognizes the extracellular domain of human, mouse and rat LDLR at 10μg/mL during 1h incubation. In C, the same antibody labels the rat RBCEC LDLR following fixation of cells with PFA 4%.

Flow cytometry (FACS): Mechanical dissociation of the RBCEC monolayer with EDTA 0.25 mM. In D, control RBCEC labelled with phycoerythrin PE-A. In E, DiI-LDL binding on the LDLR receptors of the RBCEC, at 40μg/mL during 90 min at 4°C. In F, DiI-LDL binding on RBCEC from LDLR -/- rats (LDLR knock-out, KO rats), at 40 μg/mL during 90 min at 4°C. In G, RBCEC with secondary antibody only (donkey anti goat Allophycocyanin APC, used as control). In H, labelling with a goat anti LDLR antibody at 10μg/mL during 45 min at 4°C, followed by secondary antibody. In I, labelling with the goat anti LDLR antibody on RBCEC from LDLR -/- rats at 10 μg/mL during 45 min at 4°C, followed by secondary antibody. In J, *in vitro* BBB model: rat brain capillary endothelial cells (RBCEC) co-cultured with astrocytes: transport in the lower compartment of DiI-LDL at different concentrations after 30 min incubation on live WT and LDLR -/- RBCEC.

Figure 6. *In vitro* BBB model: rat brain capillary endothelial cells (RBCEC) co-cultured with astrocytes. A to F: incubations on live RBCEC from wild type (WT) rats, G and H: live RBCEC from LDLR -/- rats. In A, binding/uptake of Fc at 0.25 μM during 30 min incubation on live WT RBCEC. In B, binding/uptake of Fc-SEQ ID NO: 43 at 0.25 μM during 30 min incubation on live WT RBCEC. In C and E, binding/uptake of DiI-LDL at 40 μg/mL during 30 min incubation on live WT RBCEC. In D, binding/uptake of Fc-SEQ ID NO: 43 at 0.25 μM during 30 min incubation on live WT RBCEC.

These photomicrographs show the co-localization between DiI-LDL and Fc-SEQ ID NO: 43. In E, binding/uptake of DiI-LDL at 40 μg/mL during 30 min incubation on live RBCEC from LDLR -/- rats. In F, binding/uptake of Fc-SEQ ID NO: 43 at 0.25 μM during 30 min incubation on live RBCEC from LDLR -/- rats. In G, binding/uptake of Fc-CTRL at 0.1 μM during 30 min incubation on live WT RBCEC. In H, binding/uptake of Fc-SEQ ID NO: 1 at 0.1 μM during 30 min incubation on live RBCEC from LDLR -/- rats.

Figure 7. *In vitro* BBB model: rat brain endothelial cells (RBCEC) co-cultured with astrocytes.

In A, binding/uptake of Fc and Fc-SEQ ID NO: 43 at different concentrations after 30 min incubation on live WT RBCEC. In B, binding/uptake of Fc-SEQ ID NO: 1 and Fc-CTRL at 0.1 μM after 2 hr incubation on live WT RBCEC.

Figure 8. Peptide vector dimer. Dimers show better affinities for LDLR than their monomer form.

In A, two examples of dimers based on two different molecular constructions. A-1: Example of dimer synthesized by peptide coupling on a triamine commercial platform. R can be a hydrogen, a tracer molecule, an active substance or any substance of interest with or without a linker in between. A-2: Example of dimer synthesized by peptide coupling on a « home-made » di-lysin platform. Ri and/or $R_2$ can be a hydrogen, a tracer molecule, an active substance or any substance of interest with or without a linker in between.

In B, quantification of binding/uptake of peptide SEQ ID NO: 1-STag to CHO-hLDLR-GFP by an ELISA anti-STag. CHO-hLDLR-GFP cells were incubated 60 min at 37°C with peptide SEQ ID NO: 1-STag alone (100% of binding) at 10 μM or with peptide SEQ ID NO: 1-STag in competition with peptide SEQ ID NO: 1, peptide CTRL or peptide D2(SEQ ID NO: $1)_2$ at 10 μM.

In C, binding of peptide D2(SEQ ID NO: $1)_2$-Cy3.5 and D2$(CTRL)_2$-Cy3.5 at 500 nM, 30 min at 4°C to CHO-hLDLR-GFP. Cell nuclei are stained with Hoechst. Photomicrographs show the co-localization between peptide D2(SEQ ID NO: $1)_2$-Cy3.5 and LDLR at the plasma membrane.

Figure 9. Tissue distribution of peptide SEQ ID NO: 1 alone or chemically coupled to the human Fc fragment in WT mice or LDLR -/- mice (KO).

In A, tissue distribution of tritium-labeled peptide SEQ ID NO: 1, 10 min following tail vein injection in WT or LDLR -/- mice. Results are expressed as percentage of total injected dose per gram tissue (%DI/g). Counting of radioactivity in tissues shows a greater accumulation of the peptide in LDLR-enriched tissues of WT compared to LDLR -/- mice. In B, tissue distribution of the chemically coupled Fc-SEQ ID NO: 1, two hours following tail vein injection in WT or LDLR -/- mice. Results are expressed as percentage of total injected dose per gram tissue (%DI/g). Quantification of the accumulation of the Fc moiety in tissues using an ELISA assay shows a greater accumulation of Fc in LDLR-enriched organs of WT compared to LDLR -/- mice.

## DETAILED DESCRIPTION OF THE INVENTION

The invention relates to peptide derivatives capable of binding human LDLR and the use thereof in the field of pharmaceuticals, notably to transport molecules of therapeutic or diagnostic interest across cell membranes of LDLR-expressing tissues (such as liver, adrenal glands or intestine), healthy or pathologic (e.g., cancer cells), and in particular across the physiological barriers BBB/BSCB and BRB. The peptides and multimers of the invention have high affinity for human LDLR and can transport molecules in vivo. The peptides or pseudo-peptides and multimers of the invention can be easily synthesized chemically, and any molecule of therapeutic or diagnostic (e.g., imaging) interest can be coupled thereto simply and effectively via a spacer (synthesis via a linker) or by direct coupling (synthesis in tandem) between the two entities (Figure 1). The peptides and pseudo-peptides are designed to adopt a cyclic configuration, thus more resistant to proteolysis. Moreover, the peptides and multimers of the invention bind LDLR without competition with the natural ligand.

The peptides or pseudo-peptides or multimers of the invention can be used as vectors for molecules of therapeutic interest, or for imaging or diagnostic agents, or for any other molecule such as a molecular probe, in the treatment, imaging and/or diagnosis of neurological pathologies, as well as genetic, infectious, inflammatory, or cancerous pathologies of the brain or other tissues.

The peptides, pseudo-peptides and multimers described in the present invention have the capacity to target cell receptors and particular cell types and organs, notably cancer cells, nervous or non-nervous tissues such as the liver, adrenal gland or intestine, and/or to cross cell membranes, notably those of the physiological barriers of the nervous system and more particularly of the CNS and eye, particularly the BBB, BSCB, BRB, or the blood-tumor barrier (BTB) of cancerous nervous tissue tumors.

The peptides, pseudo-peptides and multimers of the present invention have the capacity to bind to human, mouse and/or rat LDLR of the cell membranes of particular cell types or organs such as the liver, adrenal gland or intestine, notably those of the physiological barriers of the CNS and eye, and to cross the aforesaid membranes via this receptor by endocytosis or receptor mediated transport/transcytosis (RMT).

The peptides, pseudo-peptides and multimers may therefore be used to design drugs suitable for treating various diseases such as infectious pathologies or other pathologies of a bacterial, viral, parasitic or fungal nature/origin.

Disclosed in the invention are more particularly peptides or pseudopeptides comprising a sequence selected from SEQ ID NO: 1-5.

SEQ ID NO: 1, (D)-Cys-Met-Thz-Arg-Leu-Arg-Gly-Pen;
SEQ ID NO: 2, (D)-Cys-Met-Thz-Arg-Leu-Arg-Sar-Pen;
SEQ ID NO: 3, (D)-Cys-Met-Pip-Arg-Leu-Arg-Sar-Cys;
SEQ ID NO: 4, (D)-Cys-Met-Pip-Arg-Leu-Arg-Gly-Pen;
SEQ ID NO: 5, (D)-Cys-Met-Pip-Arg-Leu-Arg-Sar-Pen.

Further disclosed in the invention are multimeric agents of the following formula (A):

$$P_h\text{-}M\text{-}P_i \qquad (A)$$

wherein,

- each P represents the same or a different peptide or pseudopeptide comprising the amino acid sequence: A1-Met-A2-Arg-Leu-Arg-A3-A4 wherein A1 and A4 independently represent a cysteine or an analogue thereof or an isostere thereof, A2 represents a proline or an analogue thereof or an isostere thereof, and A3 represents a glycine or an analogue thereof or an isostere thereof;
- M is a Molecular platform, and
- h and i are, independently from each other, an integer selected from 1, 2, 3, 4, or 5.

A1 typically represents a residue selected from cysteine (Cys, C), of D or L configuration, or a derivative thereof selected from 2-amino-3-mercaptopropanoic acid and S-substituted derivatives thereof, S-acetylcysteine or 2-amino-3-(acetylthio)propanoic acid, selenocysteine (Sec, U) or 2-amino-3-(seleno)propanoic acid, cysteinol, 3-mercaptopropanoic acid (Mpa) or penicillamine (Pen), of L or D configuration. In a preferred embodiment, A1 is D-Cys, Pen or D-Pen.

A2 preferably represents a residue selected from proline (Pro, P) or pyrolidine-2-carboxylic acid, homoproline or 2-(2-pyrrolidinyl)ethanoic acid, 3-hydroxyproline (3Hyp), 4-hydroxyproline (4Hyp), 3-methylproline, 3,4-dehydroproline, 3,4-methanoproline, 4-aminoproline, 4-oxoproline, thioproline or thiazolidine-4-carboxylic acid (Thz), 2-oxothiazolidine-4-carboxylic acid, indolin-2-carboxylic acid (Idc), pipecolic acid (Pip) or piperidin-2-carboxylic acid, nipecotic acid (Nip) or piperidin-3-carboxylic acid, 4-oxopipecolic acid, 4-hydroxypipecolic acid, amino-1-cyclohexanecarboxylic acid, prolinol. In a preferred embodiment, A2 is selected from Pro, Pip or Thz.

A3 preferably represents a residue selected from glycine (Gly, G) or 2-aminoethanoic acid, sarcosine (Sar) or N-methylglycine (MeGly), N-ethylglycine (EtGly), allylglycine (allylGly) or 2-aminopent-4-enoic acid, 2-cyclopentylglycine (Cpg), 2-cyclohexylglycine (Chg), 2,2-dipropylglycine (Dpg), 2-(3-indolyl)glycine (IndGly), 2-indanylglycine (Igl), 2-neopentylglycine (NptGly), 2-octylglycine (OctGly), 2-propargylglycine (Pra) or 2-amino pent-4-ynoic acid, 2-phenylglycine (Phg), 2-(4-chlorophenyl)glycine, azaglycine (AzGly), or glycinol or 2-aminoethanol. In a preferred embodiment, A3 is selected from Gly and Sar.

A4 preferably represents a residue selected from cysteine (Cys, C), of D or L configuration, or a derivative thereof selected from 2-amino-3-mercaptopropanoic acid and S-substituted derivatives thereof, S-acetylcysteine or 2-amino-3-(acetylthio)propanoic acid, selenocysteine (Sec, U) or 2-amino-3-(seleno)propanoic acid, cysteinol, or penicillamine (Pen), of L or D configuration. In a preferred embodiment, A4 is selected from D-Cys, Pen, or D-Pen.

In a preferred class of multimeric agents of the invention, P designates a peptide or pseudo-peptide of formula (I'):

(D)-Cys -Met-A2-Arg-Leu-Arg-A3-A4 (I')

wherein A2, A3 and A4 are as defined above.

Particular examples of P groups are peptides comprising any one of SEQ ID NOs: 1 to 5.

The multimeric agents of the invention can comprise from 2 to 10 P groups, which may be identical or different. In a preferred embodiment, the multimeric agent is a homomultimer, i.e., all P groups are identical. Furthermore, preferred multimeric agents of the invention are dimers or trimers, even more preferably dimers. The invention indeed shows that, by using LDLR-binding peptides as defined above in a dimeric form, the binding capacity are improved and the RMT properties are increased. No such result was expected from the prior art since the natural ligand of the receptor is monomeric.

In a particular embodiment, in a multimeric agent of the invention, h is 1 or 2 and i is 1 or 2. In a more preferred embodiment, h=i=1.

The Molecular platform M may be any chemical cross-linking group compatible for use in the pharmaceutical or veterinary area. The group is preferably devoid of biological activity and toxicity. The size of the Molecular platform

may be adjusted by the skilled person. Preferred examples of M groups are polylysine platforms or multifunctional organic compounds such as Tris(2-aminoethyl)amine. Further M groups are commercially available organic compounds. In a particular embodiment, the molecular platform contains at least two reactive functional groups, preferably at least three, allowing coupling of at least 2 Peptides and at least one molecule of interest. It should be noted that the molecule of interest may be coupled either to a peptide or to the platform. Examples of reactive functional groups include, for instance, amines, acids, thiols, azides, alkynes, carbonyls, or hydrazines. In a preferred embodiment, the molecular platform is a polylyline group, preferably comprising from 2-10 lysine. Specific examples are dilysine or polylysine containing the same number of lysine residues as a peptide P. The peptides may be coupled on the platform either directly, by a covalent bond with a reactive functional group of the platform, or through a spacer, which may be composed for instance of a glycine or a series of glycine, a PEG molecule, or an aminohexanoic acid.

Particular and preferred examples of multimeric agents are molecules of the following formulas:

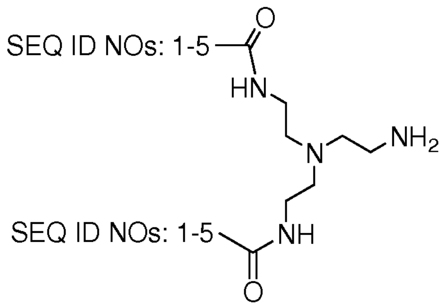

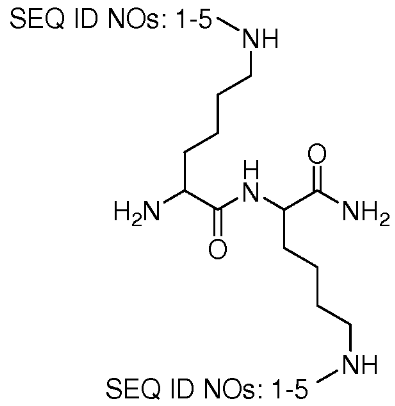

wherein "SEQ ID NOs: 1-5" designates a Peptide comprising a sequence selected from anyone of SEQ ID NOs: 1-5. It should be understood that the peptide in the above formulas may comprise any sequence of formula A1-Met-A2-Arg-Leu-Arg-A3-A4 as defined above.

The results obtained by the applicant show that the peptides and multimers of the invention have an improved affinity for LDLR. In particular, compared to a reference compound, the peptides of SEQ ID NO: 1-5 exhibit highly superior affinity, as shown in Table 1 (EXAMPLE II). In addition, the invention surprisingly shows that multimeric agents exhibit increased receptor affinity and high ability to target tissues such as the brain. More particularly, dimeric agents comprising 2 HDLR-binding peptides as defined above have a remarkably high ability to target tissues such as the brain.

These results are particularly remarkable taking into account the small size of the peptides (the peptides above contain 8 amino acids), which constitutes an additional advantage in their industrial use.

As indicated above, the peptides or pseudo-peptides or multimers of the invention can comprise peptide, non-peptide and/or modified peptide bonds. In a preferred embodiment, the peptides or pseudo-peptides comprise at least one peptidomimetic bond, chosen preferably among intercalation of a methylene ($-CH_2-$) or phosphate ($-PO_2-$) group, secondary amine (-NH-) or oxygen (-O-), alpha-azapeptides, alpha-alkylpeptides, N-alkylpeptides, phosphonamidates, depsipeptides, hydroxymethylenes, hydroxyethylenes, dihydroxyethylenes, hydroxyethylamines, retro-inverso peptides, methyleneoxy, cetomethylene, esters, phosphinates, phosphinics, phosphonamides and carba analogues.

Furthermore, in a particular embodiment, the peptides or pseudo-peptides of the invention comprise an N-term and/or C-term function respectively protected, for example, by acylation, or amidation or esterification.

The peptides or pseudo-peptides of the invention can be synthesized by any technique known to those persons skilled in the art (chemical, biological or genetic synthesis, etc.). They can be preserved as-is, or be formulated in the presence of a substance of interest or any acceptable excipient.

For chemical syntheses, commercial apparatuses that can incorporate natural as well as non-natural amino acids, such as D enantiomers and residues with side chains with hydrophobicities and steric obstructions different from those of their natural homologues (so-called exotic, *i.e.,* non-coded, amino acids), or a peptide sequence containing one or more peptidomimetic bonds that can include notably intercalation of a methylene (-CH2-) or phosphate ($-PO_2-$) group, a secondary amine (-NH-) or an oxygen (-O-) or an N-alkylpeptide, are used.

During synthesis, it is possible to introduce various chemical modifications, such as for example, putting in the N-term or C-term position or on a side chain a lipid (or phospholipid) derivative or a constituent of a liposome or a nanoparticle, in order to be able to incorporate the peptide or pseudo-peptide of the invention within a lipid membrane such as that of a liposome composed of one or more lipid layers or bilayers, or of a nanoparticle.

The peptides of the invention, or a proteic part thereof, can also be obtained from a nucleic acid sequence coding for the same. The present invention also relates to a nucleic acid molecule comprising, or consisting of, a nucleic

sequence coding for a peptide such as defined above. More particularly, the invention relates to a nucleic acid molecule comprising at least one sequence coding for a peptide of general formula (I). These nucleic acid sequences can be DNA or RNA and be combined with control sequences and/or be inserted in biological expression vectors.

The biological expression vector used is selected according to the host in which it will be transferred. It can be, for example, a plasmid, cosmid, virus, etc. The invention relates in particular to these nucleic acids and biological expression vectors, which can be used to produce the peptides of the invention, or proteic parts thereof, in a host cell. These biological expression vectors can be prepared and the peptides can be produced or expressed in a host by molecular biology and genetic engineering techniques well known to those persons skilled in the art.

The invention also discloses the use of a peptide or pseudo-peptide or multimer such as defined above, as a vector for the transfer/transport of molecules of therapeutic interest, or of imaging or diagnostic agents, or of any other molecule.

The invention also discloses use of a peptide or pseudo-peptide or multimer such as defined above for preparing a medicament capable of crossing cell membranes of LDLR-expressing tissues, healthy or pathologic, the and in particular across the physiological barrier BBB/BSCB and BRB.

The invention also discloses a method for enabling or improving the passage of a molecule across cell membranes of LDLR-expressing tissues (such as the liver, adrenal gland or intestine), healthy or pathologic (e.g., cancer cells), and in particular across the physiological barriers BBB/BSCB and BRB, comprising the coupling of the molecule to a peptide or pseudo-peptide or multimer of the invention.

The invention further discloses a conjugate compound of formula (II) as follows:

PxDy (II)

wherein P represents a peptide or pseudo-peptide comprising the amino acid sequence of anyone of SEQ ID NOs: 1 to 5, D represents an active substance or a substance of interest, and x and y are integers between 1 and 5. In a particular embodiment, x and y are equal to 1, x is greater than y, or y is greater than x.

The invention also relates to a conjugate compound of formula (III) as follows:

VxSzDy (III)

wherein V represents a peptide or pseudo-peptide or multimer of the invention, S represents a spacer, D represents an active substance or a substance of interest, x is an integer between 1 and 5 and y and z are integers between 1 and 10. In a particular embodiment, x=z=y=1 or x=z>y or y=z>x or z>x>y.

The active substance or substance of interest can be any molecule of pharmaceutical interest, notably therapeutic, a diagnostic or medical imaging agent, or a molecular probe. It can be in particular any chemical entity of biological interest such as a small chemical molecule (antibiotic, antiviral, immunomodulator, antineoplastic, anti-inflammatory, etc.), a peptide or polypeptide (such as a neuropeptide, a hormone), a protein (enzyme, notably lysosomal, hormone, cytokine, apolipoprotein, growth factor, antigen, antibody or part of an antibody), a nucleic acid (ribonucleic acid, siRNA, miRNA or deoxyribonucleic acid of human, viral, animal, eukaryotic or prokaryotic, plant or synthetic origin, etc., whose size can range from that of a single oligonucleotide to that of the genome or a genome fragment), a viral genome or a plasmid, a ribozyme, a marker or a tracer. Generally, the "substance of interest" can be any drug active ingredient, whether a chemical, biochemical, natural or synthetic compound. The expression "small chemical molecule" designates a molecule of pharmaceutical interest with a maximum molecular weight of 1000 Daltons, typically between 300 Daltons and 700 Daltons.

In the conjugate compounds of the invention, coupling between the different moieties can be carried out by any acceptable means of bonding taking into account the chemical nature, obstruction and number of associated active substances and peptides or pseudo-peptides. Coupling can thus be carried out by one or more covalent, ionic, hydrogen, hydrophobic or Van der Waals bonds, cleavable or non-cleavable in physiological medium or within cells. Furthermore, D can be coupled with V or P, if need be via S, at various reactive groups, and notably at one or more N-term and/or C-term ends of V and/or at one or more reactive groups chemically introduced or carried by the natural or non-natural amino acid side chains constitutive of V.

Coupling can be carried out at any site of the peptide or pseudo-peptide where functional groups such as -OH, -SH, $-CO_2H$, $-NH_2$, $-SO_3H$, -CN, -N3, -NCS, -PO2H, maleimide or succinimide ester are naturally present or have been introduced. Thus, a therapeutic molecule of interest, or a diagnostic (or medical imaging) agent or any other molecule such as a molecular probe can be linked (coupled) to the peptide or pseudo-peptide vector by a covalent bond either at the N-term or C-term ends, or at the reactive groups carried by the natural or non-natural amino acid side chains of this peptide sequence.

Similarly, coupling can be carried out at any site of the active substance or substance of interest (molecule of therapeutic interest, diagnostic or medical imaging agent, any other molecule such as a molecular probe) where, for

example, functional groups such as -OH,-SH, $-CO_2H$, $-NH_2$, $-SO_3H$, -CN, -N3, -NCS, -PO2H, maleimide or succinimide ester are naturally present or have been introduced.

This coupling chemistry is also suitable to conjugate Peptides or Drugs to a molecular platform as described above.

It is preferable that the interaction is sufficiently strong so that the peptide is not dissociated from the active substance before having reached its site of action. For this reason, the preferred coupling of the invention is covalent coupling, but non-covalent coupling could also be used. The substance of interest can be coupled directly to the peptide (synthesis in tandem) either at one of its terminal ends (N-term or C-term), or at a side chain of one of the constitutive amino acids of the sequence, or in case of multimers on any functional groups introduced. The substance of interest can also be coupled indirectly by means of a linker or spacer, either at one of the terminal ends of the peptides, or at a side chain of one of the constitutive amino acids of the sequence (Figure 1). Means of covalent chemical coupling, calling upon a spacer or not, include those selected from bi- or multifunctional agents containing alkyl, aryl, PEG or peptide groups by esters, aldehydes or alkyl or aryl acids, anhydride, sulfhydryl or carboxyl groups, groups derived from cyanogen bromide or chloride, carbonyldiimidazole, succinimide esters, sulfonic halides, maleimides, azide, isothiocyanate, alkynes.

In this respect, the invention also relates to a method for preparing a conjugate compound such as defined above, characterized in that it comprises a step of coupling between a peptide or multimer and a substance D, if need be via S, preferably by a chemical, biochemical or enzymatic pathway, or by genetic engineering.

The invention also relates to a pharmaceutical composition characterized in that it comprises at least one conjugate compound such as defined above and one or more pharmaceutically acceptable excipients.

The invention also relates to a diagnostic composition characterized in that it comprises a diagnostic or medical imaging agent composed of a conjugate compound such as defined above.

The conjugate can be used in the form of any pharmaceutically acceptable salt. The expression "pharmaceutically acceptable salts" refers to, for example and in a non-restrictive way, pharmaceutically acceptable base or acid addition salts, hydrates, esters, solvates, precursors, metabolites or stereoisomers, said vectors or conjugates loaded with at least one substance of interest.

The expression "pharmaceutically acceptable salts" refers to nontoxic salts, which can be generally prepared by reacting a free base with a suitable organic or inorganic acid. These salts preserve the biological effectiveness and the properties of free bases. Representative examples of such salts include water-soluble and water-insoluble salts such as acetates, N-methylglucamine ammonium, amsonates (4,4-diaminostilbene-2,2'-disulphonates), benzenesulphonates, benzonates, bicarbonates, bisulphates, bitartrates, borates, hydrobromides, bromides, buryrates, camsylates, carbonates, hydrochlorates, chlorides, citrates, clavulanates, dichlorhydrates, diphosphates, edetates, calcium edetates, edisylates, estolates, esylates, fumarates, gluceptates, gluconates, glutamates, glycolylarsanylates, hexafluorophosphates, hexylresorcinates, hydrabamines, hydroxynaphthoates, iodides, isothionates, lactates, lactobionates, laurates, malates, maleates, mandelates, mesylates, methylbromides, methylnitrates, methylsulphates, mucates, napsylates, nitrates, 3-hydroxy-2-naphthoates, oleates, oxalates, palmitates, pamoates (1,1-methylene-bis-2-hydroxy-3-naphtoates, or emboates), pantothenates, phosphates, picrates, polygalacturonates, propionates, p-toluenesulphonates, salicylates, stearates, subacetates, succinates, sulphates, sulphosalicylates, suramates, tannates, tartrates, teoclates, tosylates, triethiodides, trifluoroacetates and valerianates.

The compositions of the invention advantageously comprise a pharmaceutically acceptable vector or excipient. The pharmaceutically acceptable vector can be selected from the vectors classically used according to each mode of administration. According to the mode of administration envisaged, the compounds can be in solid, semi-solid or liquid form. For solid compositions such as tablets, pills, powders, or granules that are free or are included in gelatin capsules, the active substance can be combined with: a) diluents, for example lactose, dextrose, sucrose, mannitol, sorbitol, cellulose and/or glycine; b) lubricants, for example silica, talc, stearic acid, its magnesium or calcium salt and/or polyethylene glycol; c) binders, for example magnesium and aluminum silicate, starch paste, gelatin, tragacanth, methylcellulose, sodium carboxymethyl cellulose and/or polyvinylpyrrolidone; d) disintegrants, for example starch, agar, alginic acid or its sodium salt, or effervescent mixtures; and/or d) absorbents, dyes, flavoring agents and sweeteners. The excipients can be, for example, mannitol, lactose, starch, magnesium stearate, sodium saccharin, talc, cellulose, glucose, sucrose, magnesium carbonate and analogues of pharmaceutical quality. For semi-solid compositions such as suppositories, the excipient can, for example, be an emulsion or oily suspension, or polyalkylene glycol-based, such as polypropylene glycol. Liquid compositions, in particular injectables or those included in a soft capsule, can be prepared, for example, by dissolution, dispersion, etc., of the active substance in a pharmaceutically pure solvent such as, for example, water, physiological saline solution, aqueous dextrose, glycerol, ethanol, oil and analogues thereof.

The compositions or conjugates of the invention can be administered by any suitable route and, in a non-restrictive way, by parenteral route, such as, for example, in the form of preparations that can be injected by subcutaneous, intravenous or intramuscular route; by oral route (or *per os*), such as, for example, in the form of coated or uncoated tablets, gelatin capsules, powders, pellets, suspensions or oral solutions (one such form for oral administration can be

either with immediate release or with extended or delayed release); by rectal route such as, for example, in the form of suppositories; by topical route, in particular by transdermal route, such as, for example, in the form of patches, pomades or gels; by intranasal route such as, for example, in aerosol and spray form; by perlingual route; or by intraocular route.

The pharmaceutical compositions typically comprise an effective dose of a peptide or pseudo-peptide or conjugate of the invention. A "therapeutically effective dose" as described herein refers to the dose that gives a therapeutic effect for a given condition and administration schedule. It is typically the average dose of an active substance to administer to appreciably improve some of the symptoms associated with a disease or a pathological state. For example, in treating a cancer of the brain or of another tissue, a pathology, a lesion or a disorder of the CNS, the dose of an active substance that decreases, prevents, delays, eliminates or stops one of the causes or symptoms of the disease or disorder would be therapeutically effective.

A "therapeutically effective dose" of an active substance does not necessarily cure a disease or disorder but will provide a treatment for this disease or disorder so that its appearance is delayed, impeded or prevented, or its symptoms are attenuated, or its term is modified or, for example, is less severe, or the recovery of the patient is accelerated.

It is understood that the "therapeutically effective dose" for a person in particular will depend on various factors, including the activity/effectiveness of the active substance, its time of administration, its route of administration, its rate of elimination and its metabolism, drug combinations/interactions and the severity of the disease (or disorder) treated on a preventive or curative basis, as well as the age, weight, overall health, sex and/or diet of the patient.

Depending on the substance coupled, the conjugates and compositions of the invention can be used for treating, preventing, diagnosing or imaging numerous pathologies, notably pathologies affecting the CNS or the eye, infectious pathologies or cancers, inflammatory disease, and/or hepatic cirrhosis or fibrosis, hepatocarcinomas, diseases of other tissues such as the adrenal glands and intestine.

In this respect, the invention discloses the use of pharmaceutical conjugates or compositions as described above for treating or preventing CNS pathologies or disorders, brain tumors or other cancer cells, and bacterial, viral, parasitic or fungal infectious pathologies of the brain or other tissues, inflammatory disease, and/or hepatic cirrhosis or fibrosis, hepatocarcinomas, diseases of other tissues such as the adrenal glands and intestine.

The invention also discloses the use of the pharmaceutical conjugates or compositions as described above for diagnosing or imaging CNS or ocular pathologies or disorders, brain tumors or other cancer cells, and bacterial, viral, parasitic or fungal infectious pathologies of the brain or other tissues and inflammatory disease, and/or hepatic cirrhosis or fibrosis, hepatocarcinomas, diseases of other tissues such as the adrenal glands and intestine.

The invention also discloses the use of a conjugate or composition such as defined above for treating, imaging and/or diagnosing a brain tumor or other types of cancer cells. Studies have indeed shown that patients with certain cancers have hypocholesterolemia. This hypocholesterolemia is the consequence of an overuse of cholesterol by cancer cells. The latter to survive induce an increase in the level of LDLR expression within organs with tumors (Henricksson et al., 1989, Lancet, 2(8673), 1178-1180). There is thus a correlation between the increase in the level of LDLR expression by cells and certain cancers. It has also been recently shown that the number of LDLR is very high on the surface of certain pathological cells such as cancer cells. It is generally accepted that 1000 to 3000 LDLR are present at the surface of a non-pathological cell. Similarly, few LDLR are present in the cells of the gray matter of the cortex (Pitas et al., 1987, J. Biol. Chem., 262, 14352-14360). In the case of the glioblastoma, LDLR overexpression has been shown. Thus, on the surface of brain tumor cells, 125,000 (for U-251 cells) to 900,000 (for SF-767 cells) LDLR have been counted (Malentiska et al., 2000, Cancer Res., 60, 2300-2303; Nikanjam et al., 2007, Int. J. Pharm., 328, 86-94). It should also be noted that many tumor cells overexpress LDLR, such as those of prostate cancer (Chen et al., 2001, Int. J. Cancer, 91, 41-45), colon cancer (Niendorf et al., 1995, Int. J. Cancer, 61, 461-464), leukemia (Tatidis et al., 2002, Biochem. Pharmacol., 63, 2169-2180), colorectal cancer (Caruso et al., 2001, Anticancer Res., 21, 429-433*),* breast cancer (Graziani et al., 2002, Gynecol. Oncol., 85, 493-497), as well as cancers of the liver, adrenal glands, pancreas, ovaries, lung, etc.

The invention also discloses the use of a conjugate or composition such as defined above for treating, imaging and/or diagnosing bacterial, viral, parasitic or fungal infectious pathologies of the brain or other tissues, such as, and in a non-restrictive way, AIDS or meningitis, etc. LDLR is also present on hepatic cells. It is now known that endocytosis of the hepatitis C virus (HCV) can occur via LDLR. LDLR could serve as a viral receptor at an early stage of infection of human hepatocytes by HCV (Molina et al., 2007, J. Hepatol., 46(3), 411-419). The inventive conjugates can thus be used to specifically target pathological cells, infected by viruses such as those of hepatitis B and hepatitis C that express LDLR and/or to modulate via LDLR the viral infection process of healthy cells. The invention also relates to the use of a conjugates or composition such as defined above for treating, imaging and/or diagnosing other hepatic pathologies such as, and in a non restrictive way, non viral hepatitis provoked or not by alcohol consumption, acute hepatic inflammation, hepatic cirrhosis or fibrosis, primary biliary cirrhosis and other pathologies of the hepatobiliary tract, etc (Poeltra et al., 2012, J Control Release, 161(2):188-97).

The invention also discloses the use of a conjugate or composition such as defined above for imaging or treating diseases of the adrenal gland or intestine that are enriched in LDLR.

The invention also discloses the use of a conjugate or composition such as defined above for treating, imaging and/or diagnosing neurodegenerative pathologies such as, in a non-restrictive manner, Alzheimer's disease, Parkinson's disease, Creutzfeldt-Jakob disease, cerebrovascular accidents (CVA), bovine spongiform encephalopathy, multiple sclerosis, amyotrophic lateral sclerosis, spinal cord injuries etc.

The invention also discloses the use of a conjugate or composition such as defined above for treating, imaging and/or diagnosing neurological pathologies such as, in a non-restrictive manner, epilepsy, migraine, encephalitis, CNS pain, etc.

The invention also discloses the use of a conjugate or composition such as defined above for treating, imaging and/or diagnosing neurodegeneration and excitotoxic processes such as, in a non-restrictive manner, consequences of transient cardio-respiratory arrest, stroke, neonatal ischemia, etc.

The invention also discloses the use of a conjugate or composition such as defined above for treating, imaging and/or diagnosing neuropsychiatric pathologies such as, in a non-restrictive manner, depression, autism, anxiety, schizophrenia, etc.

The invention also discloses the use of a conjugate or composition such as defined above for treating, imaging and/or diagnosing pathologies of the eye such as, in a non-restrictive manner, retinitis pigmentosa, macular degeneration, cone-rod dystrophy (CORD), retinal separation, retinal detachment, hypertensive retinopathy and diabetic retinopathy, retinoblastoma, lipemia retinalis, etc.

The terms "treatment," "treating," "treat" and other similar expressions refer to obtaining a pharmacological and/or physiological effect, for example, inhibition of cancer cell growth, cancer cell death or improvement of a disease or neurological or ocular disorder. The effect can be prophylactic or preventive in order to completely or partially prevent the aggravation of a disease or a symptom such a disease, in an ill person, or its propagation, in healthy subjects, and/or can be therapeutic in order to completely or partially treat a disease and/or its related harmful effects. The term "treatment" as used in the present document covers any treatment of a disease in a mammal, and more particularly in man, and comprises: (a) prevention of a disease (for example, prevention of cancer) or a condition that can arise in a person predisposed to this pathology or disorder, but who has not yet been positively diagnosed, (b) the slowing of a disease (for example, by stopping its development), or (c) relief from a disease (for example, by reducing the symptoms associated with a disease). This term "treatment" also covers any administration of an active substance in order to tend, cure, relieve, improve, decrease or inhibit a condition in an individual or patient, including, but not limited to, the administration to a person in need of a drug composed of a vector or conjugate as described in the present document.

The present invention also discloses the use of a peptide or pseudo-peptide of the invention to increase the biological activity of an active substance or of a substance of interest (therapeutic molecule of interest, diagnostic or medical imaging agent, or any other molecule such as a molecular probe) to which it is coupled.

The present invention also discloses the use of a peptide or pseudo-peptide of the invention to decrease the toxicity of an active substance or of a substance of interest (therapeutic molecule of interest, diagnostic or medical imaging agent, or any other molecule such as a molecular probe) to which it is coupled.

Other aspects and advantages of the present invention will become apparent upon consideration of the examples below, which are only illustrative in nature and which do not limit the scope of the present application.

EXAMPLES

## EXAMPLE I

Synthesis of peptides and coupling with a tracer molecule (biotin, fluoresceins, rhodamines, cyanines or Stag peptide with enzymatic activity).

Peptides were synthesized by the solid phase peptide synthesis (SPPS) method on an Advanced ChemTech Apex396 (AAPPTec) synthesizer, or a Liberty™ (CEM) microwave synthesizer, using an Fmoc/tBu strategy on Rink Amide AM resin on polystyrene-1% DVB, Wang on polystyrene-1% DVB, Barlos (2-chlorotrityl chloride) on polystyrene-1% DVB, or Sieber Amide on polystyrene-1% DVB. The load (or substitution) is between 0.25 mmol/g and 1.6 mmol/g according to the resin used.

The amino acids N-protected by Fmoc (or Boc for certain N-term ends) and/or protected by orthogonal functions (notably acid labile functions) at their side chains, the chemical coupling and deprotection reagents and the solvents are bought from specialized companies and are used as-is.

The Rink Amide and Wang resins make it possible to synthesize peptide sequences completely deprotected on their side chains and their C-term ends. This is thus 2-dimension (Fmoc/tBu) orthogonal SPPS.

Barlos and Sieber hypersensitive acid labile (HAL) resins respectively enable the release of the terminal (C-term) acid or amide function while preserving the orthogonal side protections of the various amino acids of the synthesized peptide as well as the terminal (N-term) amine protection of the amine function of its last amino acid (for example, N-acetylation for questions of stability of the neosynthesized peptide sequence). These type of resins, via an Fmoc ($Prot_1$)

synthesis strategy, makes it possible to use acid-labile orthogonal side protections ($Prot_2$: Boc, tBu, OtBu, Trt, Mmt, Acm, etc.) cleavable only in strongly acid medium, whereas the protected peptide is cleaved in very weak acid conditions. This type of cleavage makes it possible to recover the peptide sequence completely protected on its side functions ($Prot_2$) with a view in particular to coupling a therapeutic molecule of interest with the peptide. This is thus three-dimension (Barlos or Sieber/Fmoc/tBu) orthogonal SPPS.

The standard orthogonal side protections ($Prot_2$) used for each amino acid during peptide synthesis are: Arg(N-Pbf), Arg(N-Pmc), Asn(N-Trt), Asp(O-tBu), Cys(S-Acm), Cys(S-Mmt), Cys(S-4MeBn), Cys(S-tBu), Cys(S-Tmob), Cys(S-Trt), Glu(O-tBu), Gln(N-Trt), His(N-Trt), Lys(N-Boc), Pen(S-Acm), Pen(S-Trt), Ser(O-tBu), Thr(O-tBu), Trp(N-Boc), Tyr(O-tBu) (Applied Biosystems, 1998, Cleavage, Deprotection, and Isolation of Peptides after Fmoc Synthesis. *Technical Bulletin*). Gly, Sar, Ala, Val, Leu, Ile, Phe, Met, Pro, Pip and Thz do not have side protections, since their respective chemical structures do not require it.

Amino acids are coupled via activation of the acid function of the n+1 amino acid using DIEA/HBTU/HOBt or DIPC/HOBt in DMF.

Deprotection of the Fmoc ($Prot_1$) group of a new amino acid thus coupled is carried out using 20% piperidin in DMF.

The last amino acid coupled during peptide sequencing will either be protected by a Boc function (with a view to releasing its free terminal amine function at the end of synthesis), or acetylated (in order to stabilize the synthesized neopeptide but also to reduce the risks of secondary reactions during covalent coupling of the therapeutic molecule of interest in the C-term position, for example) or propionylated.

According to the peptide synthesized, disulfide bridges are obtained by intramolecular cyclisation from two thiol functions of two suitably protected Cys (Acm, Trt, tBu, etc.), either in solution or on resin, using reagents classically used by those persons skilled in the art: $H_2O$/AcOH/,/$(NH_4)_2CO_3$/DMSO, $H_2O$/AcOH,/$(NH_4)_2CO_3$, $I_2$/DMF, $I_2$/HFIP/DCM, TFA/DMSO/anisole, $I_2$/DCM/MeOH/$H_2O$, etc. A Cys in the N-term position can advantageously be replaced by Pen or Mpa for cyclisation via a disulfide bridge. Lanthionine bridges (by cyclisation via dehydroalanine) or dicarba (by cyclisation via allylGly) can also be obtained by synthesis pathways known to those persons skilled in the art. A lactam bridge can be created between the side acid function of a Glu (or Asp) residue and a side amine function on a Lys or an N-term amine. Similarly, cyclisation between the N-term amine function and the C-term acid function (head/tail) can be carried out via an amide bond, just as cyclisation between the side amine function of Lys and the C-term acid function of the peptide.

Peptides from Barlos or Sieber resins are cleaved by methods classically used by those persons skilled in the art, either with 0.5% TFA (v/v) in DCM, or with AcOH/TFE/DCM (1/1/3), or with HFIP (30%) in DCM, or with TFE (30%) in DCM, etc Deprotection of side chains, and cleavage of peptides from Rink Amide or Wang resins, are carried out by methods classically used by those persons skilled in the art: either with TFA/$H_2O$/TIS or TIPS (95/2.5/2.5), or with TFA/$H_2O$/EDT/TIS or TIPS (94/2.5/2.5/1), or with TFA/thioanisole/$H_2O$ (94/5/1), or with TFA/TIS/$H_2O$/thioanisole (90/5/3/2), or with TFA/$H_2O$/phenol/thioanisole/EDT (82.5/5/5/5/2.5), etc.

Biotins, fluoresceins, (FITC), rhodamines (rhodamine RED-X), cyanines (Cy3.5) or STag (see EXAMPLE IV, below) are generally introduced in the C-term or N-term position, these tracers are sometimes couples coupled in the N-term position, according to classic synthesis and coupling methods known to those persons skilled in the art.

Peptides are isolated and purified by HPLC on a Beckman System Gold 126 apparatus with a Chromolith C18 (4.6 mm x 50 mm) or Nucleosil C18 (10 mm x 250 mm) column with, for example, a 0% to 100% acetonitrile gradient in an aqueous phase ($H_2O$ + 0.1% TFA) in 3.5 min then 100% to 0% in 1.5 min (flow rate: 1 ml/min to 5 ml/min), or on a Waters 1525 system with a Chromolith Speed ROD RP-18 (4.6 mm x 50 mm) column (stationary phase) with detection by a Waters 996 PDA detector (190 nm - 400 nm), or on a Waters Alliance 2690 system with a Chromolith Performance RP-18 (3 mm x 100 mm) column (stationary phase) with detection by a Waters 996 PDA detector (190 nm - 400 nm). UV detection is carried out at 214 nm and 254 nm.

Preparative purifications are carried out with a Waters Prep LC 4000 system with a Guard-Pak™ column (stationary phase) with Delta-Pak™ C18 cartridges (25 mm x 10 mm) with detection by a Waters 2487 Dual Wavelength Absorbance Detector.

Molecular weights are determined using an electrospray ionization (ESI) mass spectrometer in positive mode. The spectra are obtained by using a Waters Micromass Quattro Micro (quadrupole analyzer) equipped with a Waters Alliance 2690 HPLC system enabling LC-MS coupling.

The LC-MS analysis conditions used are as follows:

- Chromolith Flash C18 column (4.6 mm x 25 mm),
- 3 ml/min flow rate,
- linear gradient from 0% to 100% of B in 2.5 min (A: 0.1% $H_2O/HCO_2H$; B: 0.1% $ACN/HCO_2H$).

Mass spectra in positive electrospray mode are acquired at a flow rate of 100-200 μl/min. The data are obtained

in scan mode from 200-1700 *m/z* with 0.1 s intervals.

**EXAMPLE II**

Peptide vector design.

The capacity of the peptides to bind hLDLR was determined.

To this end, adherent and confluent CHO-hLDLR-RFP cells were cultivated in 6-well plates. Three wells of cells are used per condition.

A solution containing 10 μM of a reference peptide1 (termed "SEQ ID NO: 17", the amino acid sequence of which being disclosed as SEQ ID NO: 17 in WO2011/131896) attached to a Stag ("SEQ ID NO: 17-Stag") was prepared in HamF12-1% BSA culture medium. To this solution was added 10 μM of the peptide to evaluate (competition).

Several control solutions are also prepared:

(i) HamF12-1% BSA medium.

(ii) HamF12-1% BSA medium + 10 μM control peptide CTRL-STag (evaluation of nonspecific binding of any peptide comprising a STag).

(iii) HamF12-1% BSA medium + 10 μM peptide SEQ ID NO: 17-STag + 10 μM control peptide CTRL (evaluation of "nonspecific" competition between the peptide of interest and the control peptide CTRL).

The FRET approaches used are those described in EXAMPLE III.

The results obtained are presented in table 1 below. The table gives the results of the competitions in % of reference peptide vector (SEQ ID NO: 17-STag), affinity for hLDLR, displaced by the peptides of the invention. The larger the displacement value, the more affinity the peptide has for hLDLR. When this value is greater than 50%, the peptide has an affinity greater than that of the reference peptide 1.

Table 1

| Peptides | Displacement of the control peptide |
|---|---|
| SEQ ID NO: 1 | 100% |
| SEQ ID NO: 2 | 93% |
| SEQ ID NO: 3 | 70% |
| SEQ ID NO: 4 | 67% |
| SEQ ID NO: 5 | 100% |
| Reference Peptide 1 ("SEQ ID NO: 17-Stag") | 50% |

The results presented show that the peptides of the invention, comprising the amino acid sequence of anyone of SEQ ID NOs: 1 to 5, have highly superior affinity for the human LDLR than reference peptide 1. These results are particularly interesting considering the small size of the peptides.

In addition, the binding affinity $K_D$ of the peptides of the invention, as measured using Surface Plasmon Resonance (Biacore®), also show remarkable results. For example, the peptide of SEQ ID NO: 1 shows a low $K_D$ value of 22 nM.

Table 2

| **Test peptides** | **$K_{on}$ ($M^{-1}s^{-1}$)** | **$k_{off}$ ($s^{-1}$)** | **$K_D$ (M)** | **Rmax** |
|---|---|---|---|---|
| SEQ ID NO: 17 | 5.77E+05 | 5.05E-02 | 8.82E-08 | 20 |
| SEQ ID NO: 1 | 3.04E+06 | 6.53E-02 | 2.24E-08 | 21 |
| D1(SEQ ID NO: 17)$_2$ | - | - | < 1E-09 | - |
| D2(SEQ ID NO: 1)$_2$ | - | - | < 1E-09 | - |
| Fc-SEQ ID NO: 43 | 5.55E+03 | 7.10E-05 | 1.33E-08 | 268 |
| Fc-SEQ ID NO: 1 | 1.03E+06 | 2.20E-04 | 2.13E-10 | 186 |

*In vitro* plasma stability (i.e. half-life or $t_{1/2}$) of the peptides of the invention was also assessed. Briefly, each peptide was incubated up to 8 hours at 37°C in freshly collected Swiss (CD-I) mouse blood and the analyte was quantified in the plasma fraction at several time-points using a liquid chromatography-tandem mass spectrometry (LC-MS/MS) analytical method. The results show that all peptides of the invention show *in vitro* half-lives values of at least 3.9 hours and up to 9.2 hours.

Table 3

| Test peptides | *In vitro* half-life (h) |
|---|---|
| SEQ ID NO: 1 | 4.0 |
| SEQ ID NO: 2 | 4.9 |
| SEQ ID NO: 3 | 5.9 |
| SEQ ID NO: 4 | 3.9 |
| SEQ ID NO: 5 | 9.2 |
| Ref Peptide2 | 1.5 |
| Ref Peptide 1 | 3.0 |

Accordingly, the peptides or pseudo-peptides of the invention have the following remarkable pharmacological properties:

(i) an apparent affinity (Km) for hLDLR in the range of 100 nM and an affinity ($K_D$) in the range of 10 nM, which is highly superior to prior peptides and is adapted to the binding, endocytosis, intracellular -or post transcytosis/excretion- release of the peptide *(*Yu et al., 2011, Sci Transl Med., May 25;3(84):84ra44*)* ;
(ii) physicochemical characteristics that favor their specificity for the hLDLR (constrained conformation via disulfide bridge cyclization); a size reduced to 8 amino acids; up to 4 non-natural amino acids for promoting their specificity for this receptor;
(iii) a greater resistance to enzymatic proteolysis: while endogenous peptides and generally small linear peptides containing only natural amino acids have very short *in vitro* half-lives ($t_{1/2}$) in blood, typically in the minute range (Foltz et al., 2010, J Nutr., Jan;140(1):117-8), the $t_{1/2}$ of peptides of the invention range from 1.5 hours, up to 9.2 hours.
(iv) a small size and a molecular weight near 1 kDa making it possible to reduce the costs of synthesis and of future production on an industrial scale.

**EXAMPLE III**

Binding and endocytosis of synthesized peptides with affinity for hLDLR in CHO-LDLR-GFP cell lines.
Peptides of the invention are coupled/conjugated in the C-term or N-term position with various tracer molecules, either rhodamine Red-X, Cy3.5, FITC or STag, separated by a spacer generally composed of three Gly residues (Figure 2). The STag (a 15 amino acid peptide derived from sequence 1-15 of bovine pancreatic ribonuclease A) on the one hand can be recognized by an anti-STag antibody for immunocytochemistry or FACS approaches, and on the other hand can reconstitute enzymatic activity by binding with ribonuclease S-protein (C-term portion, amino acids 21-124) in tests of activity *in vitro* using the FRETWorks STag assay kit (Novagen 70724-3). The ribonuclease thus activated digests an RNA substrate releasing a masked fluorescent agent visualized by FRET (fluorescence resonance energy transfer) and quantified in a 96-well plate in a Beckmann spectrofluorimeter. For these FRET experiments, control CHO cells were used and GFP fused in the C-term position with hLDLR and mLDLR, which generates strong background noise at the wavelengths used for FRET, was replaced by red fluorescent protein (RFP). The stable cell lines generated for the FRET experiments are thus CHO-RFP and CHO-hLDLR-RFP.
For the FRET approaches, cells are washed twice with 2 ml PBS and then incubated for 1 h at 37 °C with 250 µl peptide solution. They are again washed twice with 2 ml PBS and then twice with 1 ml PBS, and then scraped in 1 ml PBS and centrifuged for 5 min at 1250 rpm. The supernatant is then aspirated and the cell pellet is lysed in 80 µl PBS + 0.1% Triton X100. Twenty µl of each cell lysate is analyzed by measuring fluorescence emission after the FRET reaction.
Thus, experiments involving the incubation of peptides on various cells expressing hLDLR are performed demonstrating that the peptides bind to CHO-LDLR-GFP cells and that they undergo endocytosis to accumulate in the cells of the cell line that expresses hLDLR, which is not the case for the control peptides. In these experiments, preliminary incubation of peptides conjugated with STag, with a primary antibody (primary Ab) directed against STag, and a secondary

antibody (secondary Ab) directed against the primary antibody, show that the complex between a peptide-STag, a primary Ab and a secondary Ab binds to cells expressing hLDLR and is internalized by endocytosis. These results indicate that the peptides of this invention can bind to cells expressing hLDL and vectorize large loads (two antibodies), i.e., these loads are internalized by endocytosis.

In order to further assess the precise subcellular trafficking of peptides conjugated to STag, pulse-chase experiments were performed in CHO-hLDLR-GFP cells (Figure 3). In these experiments, the complex composed of peptide SEQ ID NO: 1-STag / primary antibody /secondary antibody is incubated with CHO-hLDLR-GFP cells for 30 minutes at 4°C together with LDLs, the natural ligand of LDLR, coupled to the fluorescent probe DiI (DiI-LDL). Cells are then washed 3 times with 1 ml PBS and incubated in chase media for 5 minutes (Figure 3 - T5), allowing accumulation of cytoplasmic endocytic vesicles, or for 3 hours (Figure 3 - T180), allowing trafficking and fusion of endosomes with lysosomes in which the natural ligand LDL accumulates while the LDLR is recycled back to the plasma membrane via the recycling endosomal pathway. Cells are then fixed with PFA 4% for 10 minutes and processed without permeabilization. The results indicate that i) the peptides of the invention bind specifically hLDLR expressed at the surface of cells and undergo LDLR-mediated endocytosis without altering the binding and endocytosis of LDLs, the natural ligand of LDLR, ii) following endocytosis, a substantial part of these peptides follow the same intracellular trafficking pathway than LDLs, ultimately leading to the fusion of peptide-containing vesicles with lysosomes (Figure 3 - T180/inset 2), while another part which varies among peptide analogues follow the same recycling route than the LDLR (Figure 3 - T180/inset 1).

**EXAMPLE IV**

Toxicity, endocytosis and transcytosis of synthesized peptides with affinity for LDLR on endothelial cells in BBB *in vitro* models.

The potential toxic effects of peptides on endothelial cells, the binding/accumulation of peptides in these cells, and the passage by transcytosis of peptides are evaluated on *in vitro* BBB models. The cells necessary to set up the co-culture model are rat or mouse brain capillary endothelial cells (BCECs) and rat or mouse astrocytes. This type of *in vitro* BBB model is used to evaluate the passive passage or active transport of numerous molecules, notably pharmacological agents, across BCECs and thus by extrapolation their capacity to reach CNS tissue *in vivo.* The different models developed to date (bovine, porcine, murine, human) models have ultrastructural properties characteristic of the brain endothelium, notably tight junctions, absence of fenestrations, absence of transendothelial channels, low permeability to hydrophilic molecules and high electrical resistance. Moreover, these models have shown solid correlations between the results of measurements taken on various molecules evaluated *in vitro* and *in vivo* for their property of passing across the BBB. To date, all the data obtained show that these *in vitro* BBB models closely imitate the situation *in vivo* by reproducing some of the complexities of the cell environment that exist *in vivo,* while preserving the advantages associated with cell culture experimentation.

For example, the *in vitro* rat BBB model brings into play a co-culture of BCECs and astrocytes. Prior to cell culture, membrane inserts (Millicell 1.0 μm porosity; for 6-well or 12-well plates) are treated on the upper part with collagen type IV and fibronectin in order to enable optimal adhesion of BCECs and to create the conditions of a basal lamina. Primary cultures of mixed astrocytes are established from neonatal rat cerebral cortex (Dehouck et al., 1990, J. Neurochem., 54, 1798-1801). Briefly, after the meninges are removed, the brain tissue is passed through an 82 μm nylon sieve. The astrocytes are distributed in microplate wells at a concentration of $1.2x10^5$ cells/ml with 2 ml optimal culture medium (DMEM) supplemented with 10% heat-inactivated fetal calf serum. The medium is changed twice per week. The BCECs are grown in the presence of DMEM/F12 medium supplemented with 20% (v/v) bovine platelet poor plasma derived serum, 2 mm glutamine, 50 μg/ml gentamicin and 1 ng/ml basic fibroblast growth factor, added every two days. The BCECs are then distributed on the upper surface of the filters in 2 ml co-culture. This BCEC medium is changed three times per week. Under these conditions, differentiated BCECs form a monolayer of confluent cells seven days later.

To test their toxicity, the peptides of the invention chemically conjugated or fused to human Fc fragment of an IgG1 antibody are incubated in the upper chamber of the culture system, in contact with endothelial cells for 30 min or 2 hr. The culture medium of the lower chamber is collected at various times and Fc fragment quantified by sandwich ELISA. Lucifer Yellow (LY), a small fluorescent molecule which does not cross the BBB, is used first to evaluate the integrity of the BBB *in vitro,* in all the wells analyzed, and second for peptide co-incubation in order to evaluate the absence of toxicity of the peptides for the endothelial cells that form this BBB. The culture media is collected at different time points and fluorescence is quantified by fluorimetry. Results are expressed as endothelial surface permeability (or Pe) in $10^{-3}$ cm/min. The *in vitro* barrier is considered "permeable" or "open" if the Pe value of LY is greater than $0.6x10^{-3}$ cm/min. Transendothelial electrical resistance (TEER), measured with an ohmmeter and expressed in $ohm.cm^2$, also makes it possible to measure BBB integrity *in vitro* during tests of passage across the BBB. The quality threshold value is set at >500 $ohm.cm^2$.

The experiments carried out show an absence of toxicity of the peptides, as well as for the control peptide

used, and an absence of deleterious effects on the permeability properties of the BBB.

As a control, DiI-LDL was quantified in the lower compartment of the *in-vitro* BBB model (Figure 5-J WT curve). The graph shows a saturation of the transport at 4μg per insert. In order to confirm the LDLR specificity of the signal that was quantified with the DiI-LDL, this experiment was repeated with an *in-vitro* BBB model based on BCEC from the LDLR -/rats (KO). The graphs from Figure 5-J show that there is no DiI-LDL transport across the BCEC monolayer into the lower compartment when prepared from LDLR -/- rats (significant differences between experiments with WT and LDLR -/- rats BCEC).

The binding/uptake to LDLR at the BBB *in vitro* of an inventive peptide conjugated to the human Fc fragment (Fc-SEQ ID NO: 43 and Fc-SEQ ID NO: 1) is verified on the *in vitro* rat model described above (Figure 7). This analysis is carried out by measuring by sandwich ELISA the quantity of Fc fragment, or Fc fragment conjugated to the inventive peptide, accumulated in the endothelial cells and the receiver wells at various times (30 min to 2 hr) and concentrations (0.1 to 0.4 μM). The absence of toxicity is evaluated by measuring the integrity of the BBB in the various wells analyzed by simultaneous measurement of the level of LY that passes from one compartment to the other as a function of time.

## EXAMPLE V

Biomolecular fusion of reference peptide2 and biochemical coupling of reference peptide1 and of SEQ ID NO: 1, to the constant fragment of a human IgG1 antibody (Fc fragment) and evaluation of binding, endocytosis and RMT in a CHO hLDLR cell line and *in vitro* rat BBB model.

Some of the peptides having affinity for the hLDLR were tested for their ability to promote binding and/or endocytosis and/or transcytosis of covalently-linked Fc fragment to hLDLR expressed in cell lines or in primary endothelial cells. For this purpose, the inventors either cloned the sequence encoding the peptide SEQ ID NO: 43 in fusion with the Fc fragment, or chemically coupled peptides SEQ ID NO: 17 and SEQ ID NO: 1 to the same Fc fragment.

In order to produce the Fc fragment fused to peptide SEQ ID: 43, a plasmid construct was generated based on plasmid-pINFUSE hIgG1-Fc2 (InvivoGen), which was used as template. A mega-primer coding for Fc-SEQ ID NO: 43 was synthesized by PCR as described in WO2011/131896. The product of the PCR reaction (Fc-SEQ ID NO: 43) was purified, digested with DpnI (enzyme that digests the parental methylated DNA) and used as a mega primer in a second PCR reaction performed with the pINFUSE hIgG1-Fc2 plasmid used as matrix using the QuickChange II Site Directed Mutagenesis Kit (Agilent). After transformation of competent bacteria, isolated colonies were obtained and plasmid DNA was prepared and the cDNA construct was sequenced on both strands for verification. This vector, called pFc- SEQ ID NO: 43, allows expression of the Fc fragment fused in its N or C-terminus with the peptide SEQ ID NO: 43 after transfection of mammalian cells. Human Embryonic Kidney cells (HEK 293) were transfected using lipofectamine 2000 (Invitrogen) with plasmid pFc-SEQ ID NO: 43. Seventy two hours post-transfection, protein Fc-SEQ ID NO: 43 contained in the culture supernatant was purified using the Prosep Protein A Purification Kit (Millipore). The purified fusion protein was assayed by a sandwich ELISA test and was subsequently used to carry out tests on hLDLR CHO cells, primary endothelial cells and *in vitro* BBB models.

For chemical coupling of the peptides SEQ ID NO: 17 and SEQ ID NO: 1 to the Fc fragment (Millipore), the Controlled Protein-Protein Crosslinking Kit (Thermo Scientific) was used. The peptides SEQ ID NO: 17 and SEQ ID NO: 1 were synthesized to carry a free sulfhydryl group (cysteamide) available for linkage with a purified Fc fragment with maleimide groups added on primary amines of the Fc fragment. The number of moles of peptide per mole of Fc fragment was evaluated from 1 to 7. The obtained proteins were named Fc-SEQ ID NO: 17 and Fc-SEQ ID NO: 1 and were assayed by a sandwich ELISA test. They were subsequently used to perform tests on CHO hLDLR cells.

The binding and endocytosis of Fc-SEQ ID NO: 43 and of Fc-SEQ ID NO: 1 were also assessed by immunocytochemistry using pulse-chase experiments (Figure 4). Briefly, CHO-hLDLR-GFP cells were incubated for 30 minutes at 4°C with Fc-peptides at the concentration of 5 nM, then extensively washed and incubated for 5 minutes at 37°C in chase media to allow accumulation of cytoplasmic endocytic vesicles. Cells are then fixed with PFA 4% for 10 minutes and permeabilized with PBS/Triton X100 0.1% for 10 minutes before immunostaining with an anti-human Fc antibody conjugated to Alexa 594. The peptide SEQ ID NO: 43 fused to the Fc fragment and the peptide SEQ ID NO: 1 chemically bound to the Fc fragment undergo LDLR-mediated endocytosis as observed with peptides coupled to the STag. No binding is observed for Fc alone. No binding is observed on CHO WT cells (not shown).

The rat BCEC from the *in-vitro* BBB model as described in Example V was used in binding/uptake and transport experiments of Fc-SEQ ID NO: 43 versus Fc fragment alone used as a negative control and Fc-SEQ ID NO: 1 versus Fc-CTRL (scramble of SEQ ID NO: 43) as negative control. The expression of the LDLR and its functionality for RMT in the *in-vitro* BBB model were assessed (Figure 5). Then, Fc-SEQ ID NO: 43 versus Fc were incubated at different concentrations during 30 min on live RBCEC at 37°C (Figure 7-A). Fc-SEQ ID NO: 1 versus Fc-CTRL were also incubated at 0.1 μM during 30 min (Figure 6-G and 6-H) or 2 hr on live RBCEC at 37°C (Figure 7-B). Following this co-incubation, the cell monolayer was washed extensively and fixed with PFA at 4%. The cell monolayer was permeabilized with a solution of 0.1% Triton X100. Fc, Fc-SEQ ID NO: 43, Fc-SEQ ID NO: 1 and Fc-CTRL were revealed using a primary

antibody against the human Fc fragment (Figure 6). Then confocal microscopy was used to assess the co-localization between DiI-LDL vesicles and Fc-SEQ ID NO: 43 fluorescence (Figure 6-A to D). The same experiments were also performed with rat BCEC from LDLR -/- rats (KO) (SAGE laboratories from Sigma Aldrich) (Figure 6-E and 6-F). This experiment confirmed the role of the LDLR in DiI-LDL binding and endocytosis since DiI-LDL binding was not observed in LDLR -/- rat BCEC. Concomitantly, binding of DiI-LDL and staining for Fc-SEQ ID NO: 43 were not detected in LDLR -/- BCEC.

In order to confirm the binding/uptake of Fc-SEQ ID NO: 43 and Fc-SEQ ID NO: 1 to BCEC, different times and concentrations were assessed in the rat *in-vitro* BBB model (Figure 7). The graph in Figure 7-A shows a significant difference of binding/uptake of the Fc-SEQ ID NO: 43 in comparison with the Fc fragment. The graph in Figure 7-B shows a significant difference of binding/uptake of the Fc-SEQ ID NO: 1 in comparison with the Fc-CTRL.

**EXAMPLE VI**

Protocols for chemical synthesis of conjugates comprised of a vector and a therapeutic molecule of interest or an imaging (or diagnostic) agent or any other molecule such as a molecular probe.

A therapeutic molecule of interest or an imaging or diagnostic agent or any other molecule such as a molecular probe can be cleaved/released/salted out from the vector after transport and passage across cell membranes, more particularly the BBB, for example through a prodrug strategy by hydrolysis or enzymatic cleavage of a chemical bond between the vector and the active substance.

Covalent coupling between the peptide vector completely protected on its reactive side functions (coupling at the C-term and N-term) or partially protected (coupling on a reactive function of a side chain) and the therapeutic molecule of interest is carried out via two general strategies (Figure 1):

- synthesis in tandem (*i.e.,* direct coupling with no intermediate between the two entities),
- synthesis via a linker (Temsamani et al., 2004, Drug Discov. Today, 23, 1012-1019).

According to the peptide vector and molecule of therapeutic interest selected, one or the other of the various strategies is applied either on the C-term, or on the N-term, or on a side chain reactive function of this peptide vector. Ideally, in a prodrug strategy, the spacers selected should enable suitable release of the active substance and improvement of the solubility of the conjugate (Molema et al., 2001, Drug targeting, organ-specific strategies. In Methods and principles in medicinal chemistry, vol. 12*;* Kratz et al., 2008, Prodrug Strategies in Anticancer Chemotherapy. In ChemMedChem, vol. 3). Various labile covalent chemical bonds can thus be generated between the two entities (vector and active substance) via or not via a spacer: amides, carbamates, esters, thioester, disulfide, etc. For example, it has been shown in the literature that disulfide bonds, relatively stable in plasma, can be cleaved, by enzymes such as protein-disulfide reductase, inside the intracerebral compartment to restore a free thiol function (Saito et al., 2003, Adv. Drug Deliv. Rev., 55, 199-215).

Others compounds of interest are those wherein the spacer is a polymer such as polyethylene glycol (PEG). Indeed, it has been shown in the literature that the conjugation of an organic molecule of biological interest with PEG made it possible to increase the plasma half-life of this molecule (Greenwald et al., 2003, Adv. Drug Deliv. Rev., 55, 217-250) and to decrease its clearance.

Another strategy consists in the use of acido-unstable linkers as for example the groupings *cis*-aconityl or hydrazone, stable in the plasma environment at pH 7.4 but cleaved in the acid environment of endosomes, where the pH is situated between 5,5 and 6,5 (D'Souza and Topp, 2001, Release from Polymeric Prodrugs: Linkages and Their Degradation, In Journal of Pharmaceutical Science, vol. 93). A third family of linkers of peptidic nature that resist plasma enzymes, but highly sensitive to lysosomal proteases such as cathepsin-B, can be used, for example the tetra-peptides GFAL et GFLG (Bildstein and al., 2011, Prodrug-based Intracellular Delivery of Anticancer Agents, In Advanced Drug Delivery Review, vol. 63*).*

Vectors conjugated with an active substance or a substance of interest can be used in the diagnosis, imaging or therapy of a pathology, lesion or disorder of different cells or organs such as liver, adrenal gland and intestine, of the CNS and eye, for preparing a drug capable of crossing the BBB/BSCB/BRB, targeting a brain tumor or another type of cancer cell for preparing a drug capable of crossing cancer cell membranes, and/or infectious pathologies for preparing a drug capable of crossing cell membranes and to target the infected cells of bacterial, viral, parasitic or fungal infectious pathologies of the CNS, eye or other tissues/organs.

Examples of active substances of therapeutic interest conjugated to peptide vectors of the invention include, but are not limited to doxorubicin and other cytotoxic agents, cytarabin-C (Ara-C), ribavirin, acyclovir, adefovir and other antiviral agents, prolyl-4-hydroxylase (P4H) inhibitors, Matrix Metalloproteinases (MMPs) and other anti-fibrotic agents, etc.

**EXAMPLE VII**

Tissue distribution of peptides or peptides conjugated to drugs of therapeutic interest, imaging (or diagnostic) agents or any other substance used as a tracer.

The distribution of peptides and peptide conjugates targeting the LDLR is assessed in mice to illustrate their ability to accumulate preferentially in tissues and organs expressing high levels of the target receptor.

Similar to brain perfusion experiments, peptides and peptide conjugates injected in mice are radiolabelled using tritium. Ten minutes after intravenous injection in the tail vein of wild-type mice or mice lacking the gene encoding LDLR (LDLR-KO mice), mice are sacrificed and the radioactivity present in the plasma and tissues is quantified. The results obtained using the peptide SEQ ID NO: 1 as an example illustrate the ability of this family of peptide vectors to target the LDLR *in vivo* and to accumulate preferentially in organs and tissues that are known to express high levels of the receptor and showing high LDL uptake (i.e. high receptor activity), including the liver, adrenal glands and the intestine, with a ≈4-fold, ≈3.4-fold and ≈3.6-fold decrease in peptide concentration, respectively, in LDLR-/- (KO) mice compared to wild-type (WT) mice (Figure 9-A).

Similarly, tissue distribution of peptides fused to or chemically coupled to the human Fc fragment of an IgG1 antibody was assessed in WT or LDLR-/- mice by quantifying the Fc moiety using an ELISA assay. Two hours following intravenous injection in the tail vein, mice are sacrificed and the plasma as well as LDLR-enriched tissues are analyzed for the presence of the peptide-Fc. As an example, the results obtained with the peptide SEQ ID NO: 1 chemically coupled to Fc show that the peptides of the invention are able to trigger LDLR-dependent tissue accumulation of a coupled protein *in vivo,* with a ≈6.7-fold and ≈30-fold decrease in Fc accumulation in the liver and adrenals, respectively in LDLR-/- compared to WT mice (Figure 9-B).

**EXAMPLE VIII**

Brain perfusion *in situ* for vectors alone and vectors conjugated with a therapeutic molecule of interest or an imaging (or diagnosis) agent or any other molecule such as a molecular probe, and study of their transport kinetics across the BBB and their accumulation in the brain of mouse.

The *in situ* brain perfusion technique (in adult male OF1 mouse) is used to illustrate passage in the brain across the BBB.

Beforehand, the peptide vectors are radiolabelled with tritium ($^3H$), an element that offers the strongest sensitivity for the detection of radioactive compounds, notably on tissue sections. Radioactive peptides with high specific radioactivity (RAS, up to 100 Ci/mmol) are prepared by a strategy of acylation of the N-term amine function by tritiated propionic (or propanoic) anhydride or tritiated N-propionyl-succinimide (NPS). This tritiation method can be applied to all peptides (vectors, or conjugates between a therapeutic peptide and a peptide vector in tandem or via a linker (of peptide or organic nature), provided that modification of the N-term does not affect the affinity of the peptides for the targeted receptor (*i.e.,* LDLR) or their biological activity in the case of therapeutic peptides.

The tritiation reaction of the peptide vector in the N-term position by propionylation is carried out in DMF (1 mg peptide in 100 μl to 450 μl according to solubility) by adding 0.1 equivalent of tritiated NPS for 5 min at room temperature, then 0.9 equivalent of cold NPS (non-tritiated) for 1 h, and then a new equivalent of cold NPS for 5 h. The reaction medium is then left at 4 °C overnight and purified the following day by HPLC. The RAS for each tritiated peptide is typically between 5 Ci/mmol and 60 Ci/mmol. The total quantity of radioactivity prepared by synthesis is generally between 500 μCi and 1000 μCi.

Radiolabelled peptides (radiolabelled with $^3H$, for example) are coupled covalently with a radiolabelled active substance (radiolabelled with $^{14}C$, for example) as described for example in EXAMPLE VII. As previously mentioned, this covalent coupling is carried out according to the structure and physicochemical properties of the active substance, in particular the presence of functional chemical groups that can be modified without decreasing the biological activity of this substance. Radiolabelled conjugates are synthesized by extrapolation from synthetic pathways developed for non-radiolabelled conjugates.

The techniques briefly summarized below were developed previously to study the distribution in the brain of active substances and, in particular, the role of the BBB and, more particularly, of LDLR in the penetration of these molecules in the brain. *In situ* brain perfusion techniques are among the most technically demanding and the most difficult to perform in the mouse. However, *in situ* brain perfusion (like *in vitro* models) enables total control of the composition of the artificial perfusate in order to maintain the cells and vascularization of the brain under normal physiological and anatomical conditions within the animal, without the disrupting factor of systemic distribution.

This strategy of *in situ* brain perfusion normally carried out in the rat was adapted for mouse (Dagenais et al., 2000, J Cereb Blood Flow Metab., 20(2), 381-6) in order to broaden its application for evaluating the parameters of transport kinetics at the BBB and the blood-retinal barrier, and this also in transgenic and KO mutant mice for the receptors, enzymes or transporters of active substances. It involves catheterization of a carotid artery in anaesthetized

mice (typically OF1) and ligature of certain branches of this carotid artery (external, thyroidal, occipital) in order to specifically perfuse the internal carotid and pterygopalatine arteries, which are used to evaluate the uptake in the brain of the vectors and conjugates. The catheter makes it possible to replace general circulation by infusion with a well-controlled perfusate (bicarbonate buffer, plasma or blood) by passing by the carotid. Oxygenated Krebs bicarbonate buffer is first used to evaluate the capacities of the vectors and conjugates to pass in the brain. After catheterization of the carotid, endogenous blood flow is stopped by sectioning the ventricles of the heart in order to avoid the mixture of buffer with blood and elevation in blood pressure. The duration of the fixed flow-rate perfusion is monitored. The buffer perfusion can be extended up to 20 min, or up to 1 h in the presence of oxygen transporters (washed erythrocytes) for studies of receptor-mediated transport (RMT).

The experiments carried out made it possible to establish cerebral transport, or the transfer coefficient ($K_{in}$: relationship between distribution volume and cerebral perfusion time), of several peptide vectors and conjugates of the invention. The duration of brain perfusion for these experiments is 2-5 min with a perfusate flow rate of 2 ml/min.

For example, the Kin of the opiate peptide Dalargine, conjugated to SEQ ID NO: 17 was $\approx 20 \times 10^{-4}$ ml/s/g while the Kin of Dalargine alone was $\approx 0.9 \times 10^{-4}$ ml/s/g.

For comparison, Tf, the natural ligand of the TfR has a Kin of $3.0 \times 10^{-4}$ ml/s/g *(*Demeule et al., 2008, J. Neurochem., 106 (4), 1534-1544).

These results thus show that the conjugates of the invention, due to their small size and advantageous configuration, have a cerebral transfer coefficient greater than that of Tf.

## EXAMPLE IX

Synthesis and properties of multimers

Peptides of the invention with affinity for hLDLR-GFP/RFP were used as monomers to synthesize dimers. Monomers were coupled either in solution or on solid support on molecular platforms containing at least three reactive functional groups among amines, acids, thiols, azides, alkynes, carbonyls, hydrazines. Monomers are separated from the platform by a spacer generally composed of one glycine but it can be a PEG molecule, aminohexanoic acid or a series of glycine. Platforms can be organic commercial compounds or case by case designed using peptide chemistry.

Dimers of the invention were subsequently coupled/conjugated to various tracer or active substances using e.g., free reactive functional groups of the platform, separated or not by a spacer group such as PEGs, aminohexanoic acid, series of glycine or commercial heterobifunctional linkers.

Analytical and preparative RP-HPLC were performed using a Dionex device (Ultimate 3000™). C18 analytical and preparative columns were purchased from Phenomenex. MALDI-TOF-TOF MS was performed using an Ultraflex II Bruker, with α-cyano-4-hydroxycinnamic acid (CHCA) as matrix. A Liberty™ (CEM) microwave synthesizer was used for automated peptide synthesis.

- Synthesis of D1 dimers with P peptides: (P-CH2CH2)2-N-CH2CH2-NH2

Tris(2-aminoethyl)amine and dry DMF (Dimethylformamide) were purchased from Sigma-Aldrich, PyBop (benzotriazol-1-yl-oxytripyrrolidinophosphonium hexafluorophosphate) and DIEA (Diisopropylethylamine) from Iris Biotech.

Peptides of the invention P (2eq) were pre-activated with PyBop and DIEA (P/PyBop/DIEA: 1/1/2) 5 minutes at room temperature (RT) in dry DMF and then added to a Tris(2-aminoethyl)amine solution in dry DMF at 0°C. The reaction mixture was then allowed to stir overnight at RT. The reaction completion was monitored by analytical RP-HPLC chromatography. Pure dimer was obtained as a white powder by RP-HPLC purification. The crude dimer was purified by RP-HPLC chromatography. Collected fractions were lyophilized to give a pure white solid (2.9mg, 7% in relation to P, purity > 95%). The mass was confirmed by MALDI-TOF MS: m/z [M+H]+ calculated 2293.03 found 2294.20.

- Synthesis of D2 dimers with P peptides: protocol examples with Pr-K(P)-K(P)-NH2, Cya3.5-PEG2-K(P)-K(P)-NH2

Fmoc-Lys(Boc)-OH, Fmoc-PEG2-OH, TFA (Trifluoroacetic acid), TIS (Triisopropylsilane), HBTU (2-(1H-benzotriazole-1-yl)-1,1,3,3-tétraméthyluronium hexafluorophosphate), HATU (1-[Bis(dimethylamino)methylene]-1H-1,2,3-triazolo[4,5-β]pyridinium 3-oxid hexafluorophosphate), Piperidine, PyBop, DIEA and Fmoc-Rink Amide AM resin were purchased from Iris Biotech. Cya 3.5 and Pr2O (propionic anhydride) were purchased respectively from Interchim and Sigma Aldrich. DCM (Dichloromethane) and DMF were purchased from Analytic Lab.

Step 1: di-lysin platforms Pr-K-K-NH2 and Cya3.5-PEG2-K-K-NH2 were synthesized using Solid Phase Peptide Synthesis (SPPS). SPPS was carried out manually or on a peptide synthesizer using Fmoc (9-fluorenylmethyloxycarbonyl) amino terminus protection. The peptide was synthesized on a 100 μmol scale using a 5 fold molar excess of Fmoc-amino-acids in case of automated synthesis (0.2M) and 2.5 fold molar excess in case of manual synthesis (0.3-0.5M).

Coupling was performed on Rink Amide AM resin on polystyrene-1% DVB (loading = 0.714mmol/g) for amide C-terminal using 1:1:2 amino acid/HBTU or HATU/DMF. Deprotection was carried out using 20% piperidine/DMF. The resin bound peptide Pr-K-K-resin was obtained after N-ter propionylation using $Pr_2O$/DCM: 1/1. The resin bound peptide Cya3.5-PEG2-K-K-resin was obtained after N-ter coupling of Cya3.5-succinimide ester (1.25 eq) in DMF. Resin-bound products were cleaved using a solution comprised of TFA/TIS/H2O: 95/2.5/2.5 for 1H30 at RT. Crude peptides were precipitated using ice-cold ether, centrifuged at 3000rpm for 8min, lyophilized in H2O/0.1%TFA. White solids were obtained without purification with purity above 95%.

Step 2: peptides P were then conjugated to the peptidic platforms via lysin amino side chains in solution. Peptides P, PyBop and DIEA (P/PyBop/DIEA: 1/1/10) were added to a solution of the peptidic platform in dry DMF. The reaction mixture was then allowed to stir at RT. Reaction completion was monitored by analytical RP-HPLC chromatography. A few minutes were usually enough for the platform to completely react. Crude dimers were purified by RP-HPLC chromatography. Fractions above 95% were collected and lyophilized to give pure white solids. Pr-K(P)-K(P)-NH2: m=3.7mg, 34%, m/z [M+H]+ calculated 2476.12, found 2477.09. Cya-PEG2-K(P)-K(P)-NH2: m=1.4mg, 24% m/z [M+H]+ calculated 3104.47, found 3107.25.

Dimer peptides D2(SEQ ID NO: $1)_2$ and D2$(CTRL)_2$ were conjugated with Cy3.5 (Figure 7-A).

In order to assess the hLDLR binding ability of peptides D2(SEQ ID NO: $1)_2$ -Cy3.5 and D2$(CTRL)_2$-Cy3.5, pulse experiments were performed in CHO-hLDLR-GFP (Figure 7-B). In these experiments, the binding of 500 nM of peptides is performed for 30 min at 4°C. Cells are then washed 3 times with PBS and fixed with PFA 4%. Cell nuclei are stained in blue with Hoechst. The results indicate that peptide D2(SEQ ID NO: $1)_2$ -Cy3.5 binds to and co-localizes with the hLDLR expressed at the plasma membrane of CHO-hLDLR-GFP cells whereas peptide D2$(CTRL)_2$-Cy3.5 does not.

The binding/uptake of peptide SEQ ID NO: 1-STag versus peptide SEQ ID NO: 1, peptide D2(SEQ ID NO: $1)_2$ and peptide D2$(CTRL)_2$ in CHO-hLDLR-GFP cells was evaluated using an anti-STag ELISA test. As for the FRET approaches, cells were washed twice with 2 ml PBS and then incubated for 1 h at 37 °C with 250 μl peptide solution (10μM). Twenty μl of each cell lysate were analyzed by a sandwich ELISA test developed for S-Tag detection. Figure 12-C shows that peptide SEQ ID NO: 1-STag is slightly displaced by the CTRL peptide, half displaced by peptide SEQ ID NO: 1 and nearly totally displaced by peptide D2(SEQ ID NO: $1)_2$. The affinity of peptide D2(SEQ ID NO: $1)_2$ for LDLR is higher than that of peptide SEQ ID NO: 1.

The affinity ($K_D$) of peptide D1(SEQ ID NO: $17)_2$ and peptide D2(SEQ ID NO: $1)_2$ for hLDLR has been determined by Surface Plasmon Resonance on a Biacore system. For these experiments, a NiHc chip (XanTec ref: SCBS NIHC1000M) was conjugated with the recombinant extracellular LDLR domain (Sino Biological Inc ref: 10231-H08H). Peptides D1(SEQ ID NO: $17)_2$ and D2(SEQ ID NO: 1) have a very high affinity, below 1E-09 nM (Table 2).

**Claims**

1. A peptide selected from anyone of SEQ ID NOs: 1-3 and 5.

2. A multimeric agent having the following formula (A):

$$P_h\text{-}M\text{-}P_i \qquad (A)$$

wherein,

- each P represents the same or a different peptide of sequence: A1-Met-A2-Arg-Leu-Arg-A3-A4 wherein A1 and A4 independently represent a cysteine or an analogue thereof selected from (D)-cys, Penicillamine (Pen) and (D)-penicillamine ((D)-Pen), A2 represents a proline or an analogue thereof selected from pipecolic acid (Pip) and thiazolidine-4-carboxylic acid (Thz), and A3 represents a glycine or a Sarcosin (Sar);
- M is a cross-linking group, and
- h and i are, independently from each other, an integer selected from 1, 2, 3, 4, or 5.

3. The multimeric agent of claim 2, wherein A1 is (D)-cys.

4. The multimeric agent of any one of claims 2 or 3, wherein A2 is Pip or Thz.

5. The multimeric agent of any one of claims 2 to 4, wherein h and i are, independently from each other, 1 or 2, preferably h=i=1.

6. The multimeric agent of any one of claims 2 to 5, wherein P is a peptide of formula (I'):

(D)-Cys-Met-A2-Arg-Leu-Arg-A3-A4 (I')

wherein A2 represents Pro, Pip or Thz, A3 represents Gly or Sar and A4 represents Cys, (D)-Cys, Pen or (D)-Pen.

7. The multimeric agent of any one of claims 2 to 6, wherein P is a peptide selected from anyone of SEQ ID NOs: 1 to 5.

**8.** The multimeric agent of any one claims 2 to 7, wherein M is polylysine or a Tris(2-aminoethyl)amine group.

**9.** The multimeric agent of any one claims 2 to 8, which is a homodimer.

**10.** The peptide or multimeric agent of any one of the preceding claims, wherein said peptide contains at least one peptidomimetic bond, chosen from intercalation of a methylene ($-CH_2-$) or phosphate ($-PO_2-$) group, secondary amine (-NH-) or oxygen (-O-), alpha-azapeptides, alpha-alkylpeptides, N-alkylpeptides, phosphonamidates, depsipeptides, hydroxymethylenes, hydroxyethylenes, dihydroxyethylenes, hydroxyethylamines, retro-inverso peptides, methyleneoxy, cetomethylene, esters, phosphinates, phosphinics, phosphonamides or carba groups.

**11.** The peptide or multimeric agent of any one of the preceding claims, wherein the N-terminal (N-term) function of said peptide is protected by acylation and/or the C-terminal (C-term) function of said peptide is protected by amidation or esterification.

**12.** A conjugated compound of following formula (III):

VxSzDy (III)

wherein V represents a peptide or multimeric agent of any one of the preceding claims, S represents a spacer, D represents an active substance or a substance of interest, x is an integer between 1 and 5 and y and z are integers between 1 and 10.

**13.** The conjugated compound of claim 12, **characterized in that** x=z=y=1; x=z>y, y=z>x or z>x>y.

**14.** The conjugated compound of claim 12, **characterized in that** the active substance or substance of interest is a molecule of therapeutic interest, a diagnostic or medical imaging agent, a molecular probe, a small chemical molecule, a peptide or polypeptide, a protein, an antigen, an antibody or part of an antibody, a nucleic acid or an oligonucleotide, an siRNA, an miRNA, a ribozyme, a marker or a tracer.

**15.** The conjugated compound of claim 12, **characterized in that** the coupling between V and D, or between V and S on the one hand and S and D on the other, is carried out by one or more covalent, ionic, hydrogen, hydrophobic or Van der Waals bonds, cleavable or not in physiological medium or within cells.

**16.** The conjugated compound of claim 12, wherein D is coupled with V, if necessary via S, at one or both of the N-term and/or C-term ends of V and/or at one or more reactive groups carried by the side chains of the natural or non-natural amino acids constitutive of V.

**17.** A conjugated compound of following formula (IV):

PxDy (IV)

wherein P represents a peptide of claim 1, D represents an active substance or a substance of interest, x and y are integers between 1 and 5.

**18.** A method for preparing a conjugated compound, **characterized in that** it comprises a step of coupling a peptide or multimer of any one of claims 1 to 10 and a substance D, if necessary via a spacer S, by chemical, biochemical or enzymatic route or by genetic engineering.

**19.** A pharmaceutical composition comprising at least one conjugated compound of claim 12 or 17 and one or several pharmaceutically acceptable excipients.

**20.** A diagnostic composition comprising a diagnostic or medical imaging agent constituted of a conjugated compound of claim 12 or 17.

**Patentansprüche**

**1.** Ein Peptid ausgewählt aus einer der SEQ ID NOs: 1-3 und 5.

**2.** Ein multimeres Mittel, das die folgende Formel (A) aufweist:

$P_h$-M-$P_i$ (A)

wobei

- jedes P dasselbe oder ein anderes Peptid mit der Sequenz: A1-Met-A2-Arg-Leu-Arg-A3-A4 darstellt, wobei A1 und A4 unabhängig ein Cystein oder ein Analogon davon ausgewählt aus (D)-Cys, Penicillamin (Pen) und (D)-Penicillamin ((D)-Pen) darstellt, A2 ein Prolin oder ein Analogon davon ausgewählt aus Pipecolinsäure (Pip) und Thiazolidin-4-carbonsäure (Thz) darstellt und A3 ein Glycin oder ein Sarcosin (Sar) darstellt;
- M eine Vernetzungsgruppe ist, und
- h und i unabhängig voneinander eine ganze Zahl ausgewählt aus 1, 2, 3, 4 oder 5 sind.

**3.** Das multimere Mittel nach Anspruch 2, wobei A1 (D)-Cys ist.

**4.** Das multimere Mittel nach einem der Ansprüche 2 oder 3, wobei A2 Pip oder Thz ist.

**5.** Das multimere Mittel nach einem der Ansprüche 2 bis 4, wobei h und i unabhängig voneinander 1 oder 2 sind, vorzugsweise h=i=1.

**6.** Das multimere Mittel nach einem der Ansprüche 2 bis 5, wobei P ein Peptid der folgenden allgemeinen Formel (I') ist:

(D)-Cys-Met-A2-Arg-Leu-Arg-A3-A4 (I')

wobei A2 ein Pro, Pip oder Thz darstellt, A3 ein Gly oder Sar darstellt und A4 ein Cys, (D)-Cys, Pen oder (D)-Pen darstellt.

**7.** Das multimere Mittel nach einem der Ansprüche 2 bis 6, wobei P ein Peptid ausgewählt aus einer der SEQ ID NOs: 1 bis 5 ist.

**8.** Das multimere Mittel nach einem der Ansprüche 2 bis 7, wobei M Polylysin oder eine Tris(2-aminoethyl)amingruppe ist.

**9.** Das multimere Mittel nach einem der Ansprüche 2 bis 8, welches ein Homodimer ist.

**10.** Das Peptid oder multimere Mittel nach einem der vorhergehenden Ansprüche, wobei das Peptid mindestens eine peptidomimetische Bindung enthält, ausgewählt aus der Interkalation einer Methylen ($-CH_2-$)- oder Phosphat ($-PO_2-$)-Gruppe, eines sekundären Amins (-NH-) oder Sauerstoffs (-0-), von alpha-Azapeptiden, alpha-Alkylpeptiden, N-Alkylpeptiden, Phosphonamidaten, Depsipeptiden, Hydroxymethylenen, Hydroxyethylenen, Dihydroxyethylenen, Hydroxyethylaminen, Retro-Inversopeptiden, Methylenoxy-, Cetomethylen-, Estern, Phosphinaten, Phosphiniden, Phosphonamiden oder Carba-Gruppen.

**11.** Das Peptid oder multimere Mittel nach einem der vorhergehenden Ansprüche, wobei die N-terminale (N-Term)-Funktion des Peptids durch Acylierung und/oder die C-terminale (C-term)-Funktion des Peptids durch Amidierung oder Veresterung geschützt ist.

**12.** Eine konjugierte Verbindung der folgenden Formel (III):

VxSzDy (III)

wobei V ein Peptid oder multimeres Mittel nach einem der vorhergehenden Ansprüche darstellt, S einen Spacer darstellt, D eine aktive Substanz oder eine Substanz von Interesse darstellt, x eine ganze Zahl zwischen 1 und 5 ist und y und z ganze Zahlen zwischen 1 und 10 sind.

**13.** Die konjugierte Verbindung nach Anspruch 12, **dadurch gekennzeichnet, dass** x=z=y=1; x=z>y, y=z>x oder z>x>y ist.

**14.** Die konjugierte Verbindung nach Anspruch 12, **dadurch gekennzeichnet, dass** die aktive Substanz oder Substanz

von Interesse ein Molekül von therapeutischen Interesse, ein diagnostisches oder bildgebendes Mittel, eine molekulare Sonde, ein kleines chemisches Molekül, ein Peptid oder Polypeptid, ein Protein, ein Antigen, ein Antikörper oder ein Teil eines Antikörpers, eine Nukleinsäure oder ein Oligonukleotid, eine siRNA, eine miRNA, ein Ribozym, ein Marker oder ein Tracer ist.

**15.** Die konjugierte Verbindung nach Anspruch 12, **dadurch gekennzeichnet, dass** die Kopplung zwischen V und D oder zwischen V und S auf der einen Seite und S und D auf der anderen Seite durch ein oder mehrere kovalente, ionische, Wasserstoff-, hydrophobe oder Van der Waals-Bindungen, spaltbar oder nicht im physiologischen Medium oder innerhalb von Zellen durchgeführt wird.

**16.** Die konjugierte Verbindung nach Anspruch 12, wobei D mit V, falls notwendig über S, an einem oder beiden der N-terminalen und/oder C-terminalen Enden von V gekoppelt ist und/oder an eine oder mehrere reaktiven Gruppen, die von den Seitenketten der natürlichen oder nicht natürlichen Aminosäuren, die für V konstitutiv sind, getragen werden.

**17.** Eine konjugierte Verbindung nach folgenden Formel (IV):

PxDy (IV)

wobei P ein Peptid nach Anspruch 1 darstellt, D eine aktive Substanz oder eine Substanz von Interesse darstellt, x und y ganze Zahlen zwischen 1 und 5 sind.

**18.** Ein Verfahren zur Herstellung einer konjugierten Verbindung, **dadurch gekennzeichnet, dass** es einen Schritt des Koppelns eines Peptids oder Multimers nach einem der Ansprüche 1 bis 10 und einer Substanz D, gegebenenfalls über einen Spacer S, auf einem chemischen, biochemischen oder enzymatischen Weg oder durch Gentechnik umfasst.

**19.** Eine pharmazeutische Zusammensetzung, umfassend mindestens eine konjugierte Verbindung nach Anspruch 12 oder 17 und einen oder mehrere pharmazeutisch verträgliche Hilfsstoffe.

**20.** Eine diagnostische Zusammensetzung, umfassend ein diagnostisches oder medizinisches bildgebendes Mittel, das aus einer konjugierten Verbindung nach Anspruch 12 oder 17 gebildet wird.

## Revendications

**1.** Peptide choisi parmi l'un quelconque de SEQ ID NOs: 1-3 et 5.

**2.** Agent multimère ayant la formule suivante (A) :

Ph-M-Pi (A)

dans laquelle,

- chaque P représente un peptide identique ou différent de séquence: A1-Met-A2-Arg-Leu-Arg-A3-A4 où A1 et A4 représentent indépendamment une cystéine ou un analogue de celle-ci choisi parmi (D)-cys, Pénicillamine (Pen) et (D)-Pénicillamine ((D) -Pen), A2 représente une proline ou un analogue de celle-ci choisi parmi l'acide pipécolique (Pip) et l'acide thiazolidine-4-carboxylique (Thz), et A3 représente une glycine ou une sarcosine (Sar);
- M est un groupe de réticulation, et
- h et i sont, indépendamment l'un de l'autre, un entier choisi parmi 1, 2, 3, 4 ou 5.

**3.** Agent multimère selon la revendication 2, dans lequel A1 est (D)-cys.

**4.** Agent multimère selon l'une quelconque des revendications 2 ou 3, dans lequel A2 est Pip ou Thz.

**5.** Agent multimère selon l'une quelconque des revendications 2 à 4, dans lequel h et i sont, indépendamment l'un de l'autre, 1 ou 2, de préférence h = i = 1.

6. Agent multimère selon l'une quelconque des revendications 2 à 5, dans lequel P est un peptide de formule (I'):

(D) -Cys-Met-A2-Arg-Leu-Arg-A3-A4 (I')

dans laquelle A2 représente Pro, Pip ou Thz, A3 représente Gly ou Sar et A4 représente Cys, (D)-Cys, Pen ou (D)-Pen.

7. Agent multimère selon l'une quelconque des revendications 2 à 6, dans lequel P est un peptide choisi parmi l'un quelconque de SEQ ID NOs: 1 à 5.

8. Agent multimère selon l'une quelconque des revendications 2 à 7, dans lequel M est une polylysine ou un groupe Tris (2-aminoéthyl) amine.

9. Agent multimère selon l'une quelconque des revendications 2 à 8, qui est un homodimère.

10. Peptide ou agent multimère selon l'une quelconque des revendications précédentes, dans lequel ledit peptide contient au moins une liaison peptidomimétique, choisie parmi l'intercalation d'un groupe méthylène (-CH2-) ou phosphate (-PO2-), amine secondaire (-NH-) ou oxygène (-O-), les alpha-azapeptides, les alpha-alkylpeptides, les N-alkylpeptides, les phosphonamidates, les depsipeptides, les hydroxyméthylènes, les hydroxyéthylènes, les dihydroxyéthylènes, les hydroxyéthylamines, les rétro-inverso peptides, les méthylènoxy, les cétoéthylène, les cétoéthylène, les esters, les phosphiniques, les phosphonamides ou les groupes carba.

11. Peptide ou agent multimère selon l'une quelconque des revendications précédentes, dans lequel la fonction N-terminale (N-ter) dudit peptide est protégée par une acylation et / ou la fonction C-terminale (C-ter) dudit peptide est protégée par amidation ou estérification.

12. Composé conjugué de formule (III) suivante:

VxSzDy (III)

dans laquelle V représente un peptide ou un agent multimère selon l'une quelconque des revendications précédentes, S représente un espaceur, D représente une substance active ou une substance d'intérêt, x est un entier compris entre 1 et 5 et y et z sont des entiers compris entre 1 et 10..

13. Composé conjugué selon la revendication 12, **caractérisé en ce que** x = z = y= 1; x = z> y, y = z> x ou z> x> y.

14. Composé conjugué selon la revendication 12, **caractérisé en ce que** la substance active ou la substance d'intérêt est une molécule d'intérêt thérapeutique, un agent de diagnostic ou d'imagerie médicale, une sonde moléculaire, une petite molécule chimique, un peptide ou un polypeptide, une protéine, un antigène, un anticorps ou une partie d'un anticorps, un acide nucléique ou un oligonucléotide, un siRNA, un miARN, un ribozyme, un marqueur ou un traceur.

15. Composé conjugué selon la revendication 12, **caractérisé en ce que** le couplage entre V et D, ou entre V et S d'une part et S et D d'autre part, est réalisé par une ou plusieurs liaisons covalentes, ioniques, hydrogènes, hydrophobes ou de Van der Waals, clivables ou non en milieu physiologique ou au sein de cellules.

16. Composé conjugué selon la revendication 12, dans lequel D est couplé à V, si nécessaire par l'intermédiaire de S, à l'une ou aux deux extrémités N-ter et/ou C-ter de V et/ou à un ou plusieurs groupes réactifs portés par les chaînes latérales des acides aminés naturels ou non constitutifs de V.

17. Composé conjugué de formule (IV) suivante:

PxDy (IV)

dans laquelle P représente un peptide selon la revendication 1, D représente une substance active ou une substance d'intérêt, x et y sont des entiers compris entre 1 et 5.

18. Procédé de préparation d'un composé conjugué, **caractérisé en ce qu'**il comprend une étape de couplage d'un peptide ou multimère selon l'une quelconque des revendications 1 à 10 et d'une substance D, le cas échéant par

l'intermédiaire d'un espaceur S, par voie chimique, biochimique ou enzymatique ou par génie génétique.

**19.** Composition pharmaceutique comprenant au moins un composé conjugué selon la revendication 12 ou 17 et un ou plusieurs excipients pharmaceutiquement acceptables.

**20.** Composition de diagnostic comprenant un agent de diagnostic ou d'imagerie médicale constitué d'un composé conjugué selon la revendication 12 ou 17.

Figure 1

a). Synthesis in tandem

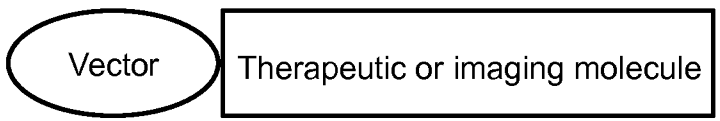


b). Synthesis via a *linker/spacer*

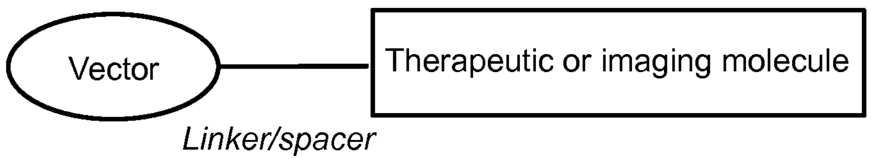

Figure 2

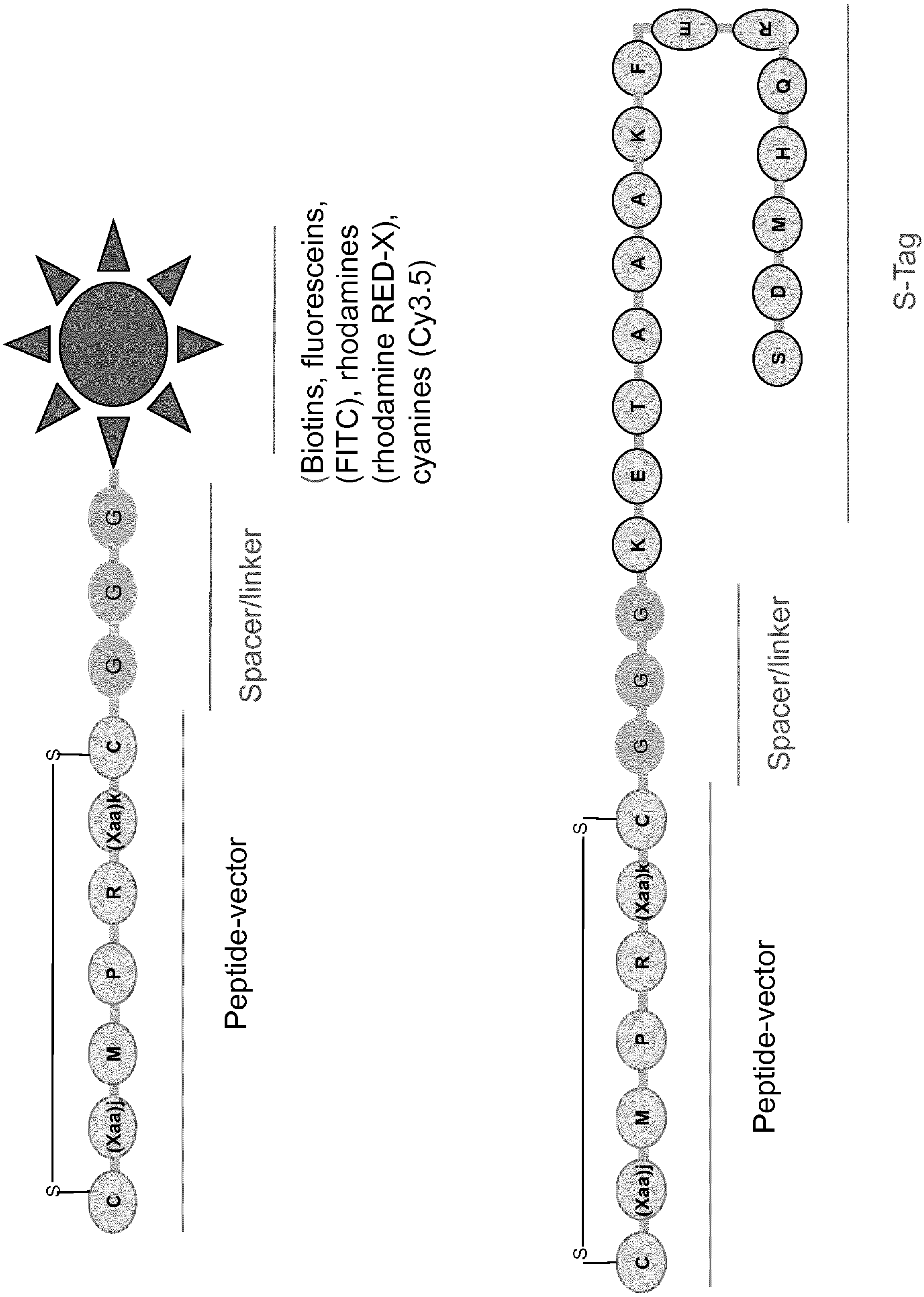
Peptide-vector
Spacer/linker
(Biotins, fluoresceins, (FITC), rhodamines (rhodamine RED-X), cyanines (Cy3.5)
Peptide-vector
Spacer/linker
S-Tag

Figure 3

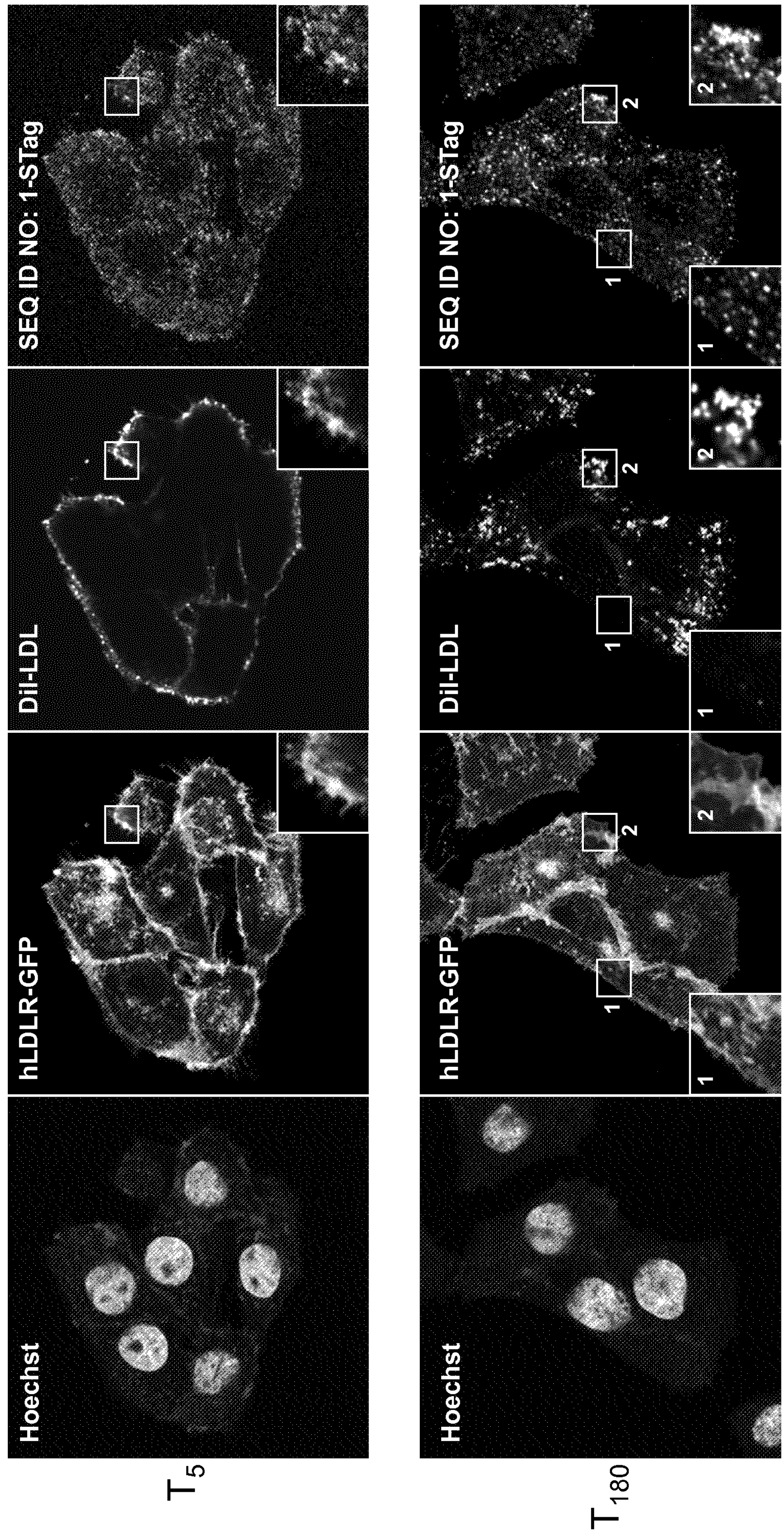

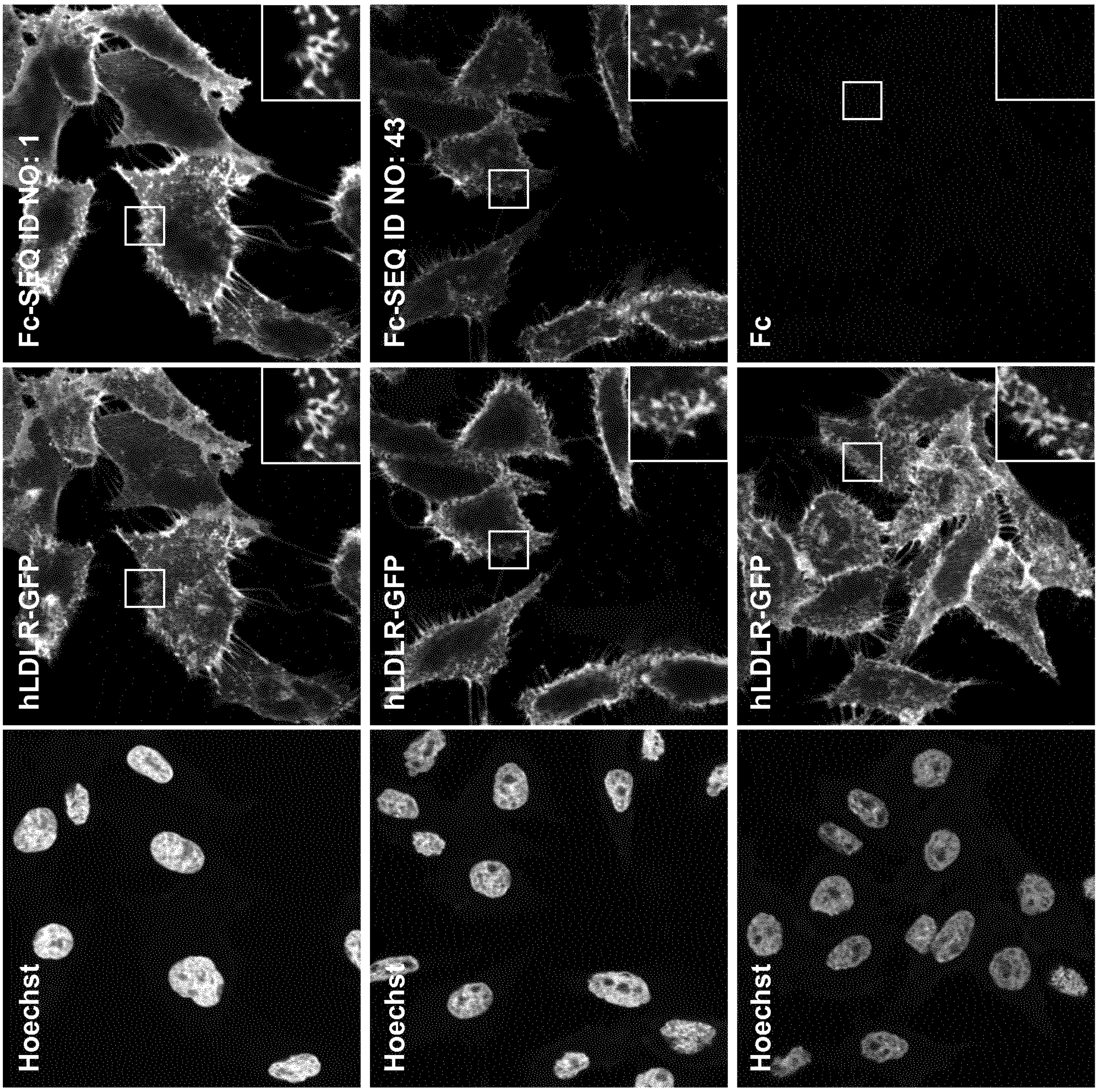
Fc-SEQ ID NO: 1
hLDLR-GFP
Hoechst
Fc-SEQ ID NO: 43
hLDLR-GFP
Hoechst
Fc
hLDLR-GFP
Hoechst

Figure 4

Figure 5

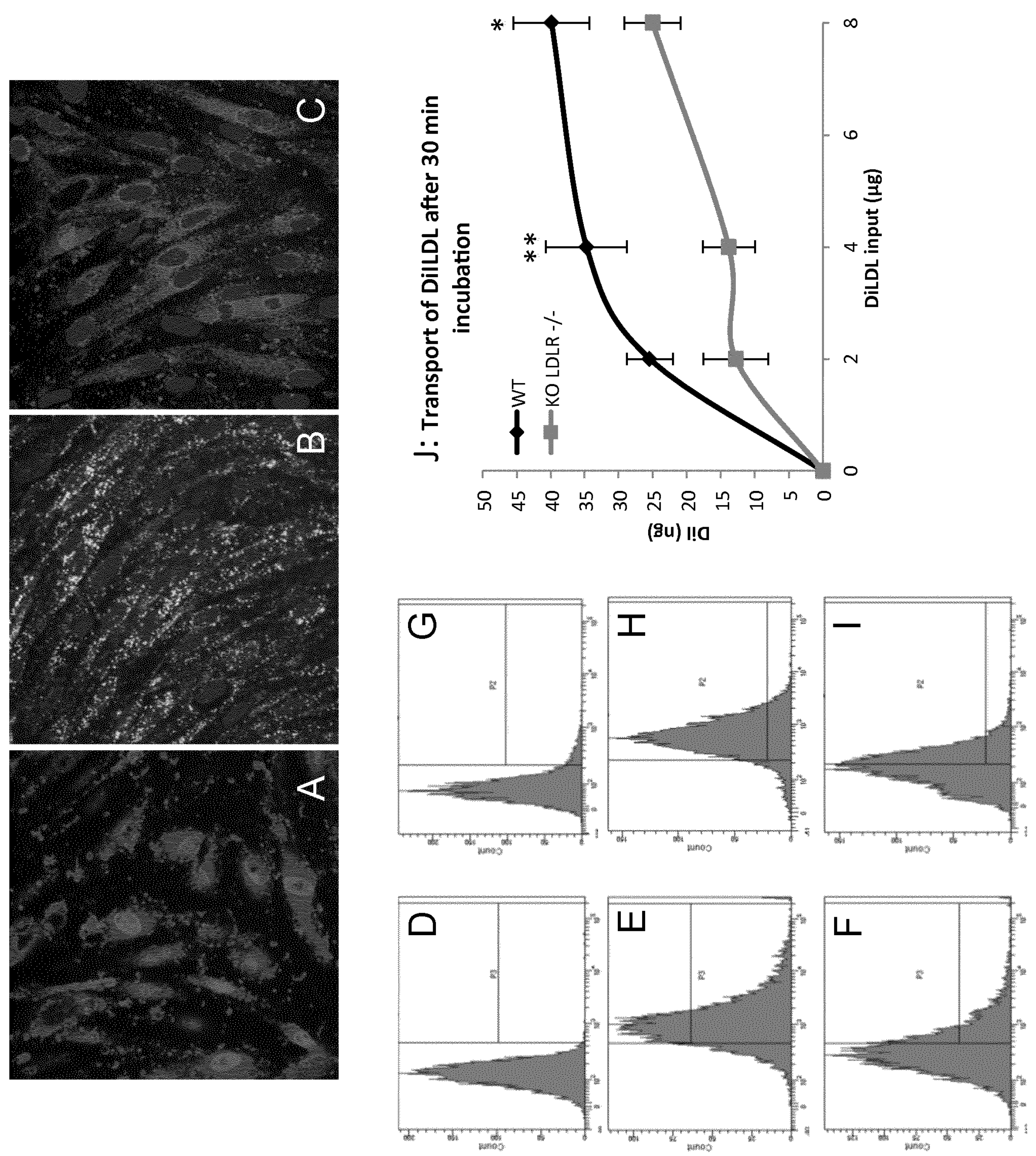
A
B
C
D
E
F
G
H
I
J: Transport of DiILDL after 30 min incubation
WT
KO LDLR -/-
DiI (ng)
DiLDL input (µg)
0
5
10
15
20
25
30
35
40
45
50
2
4
6
8
**
*

Figure 6

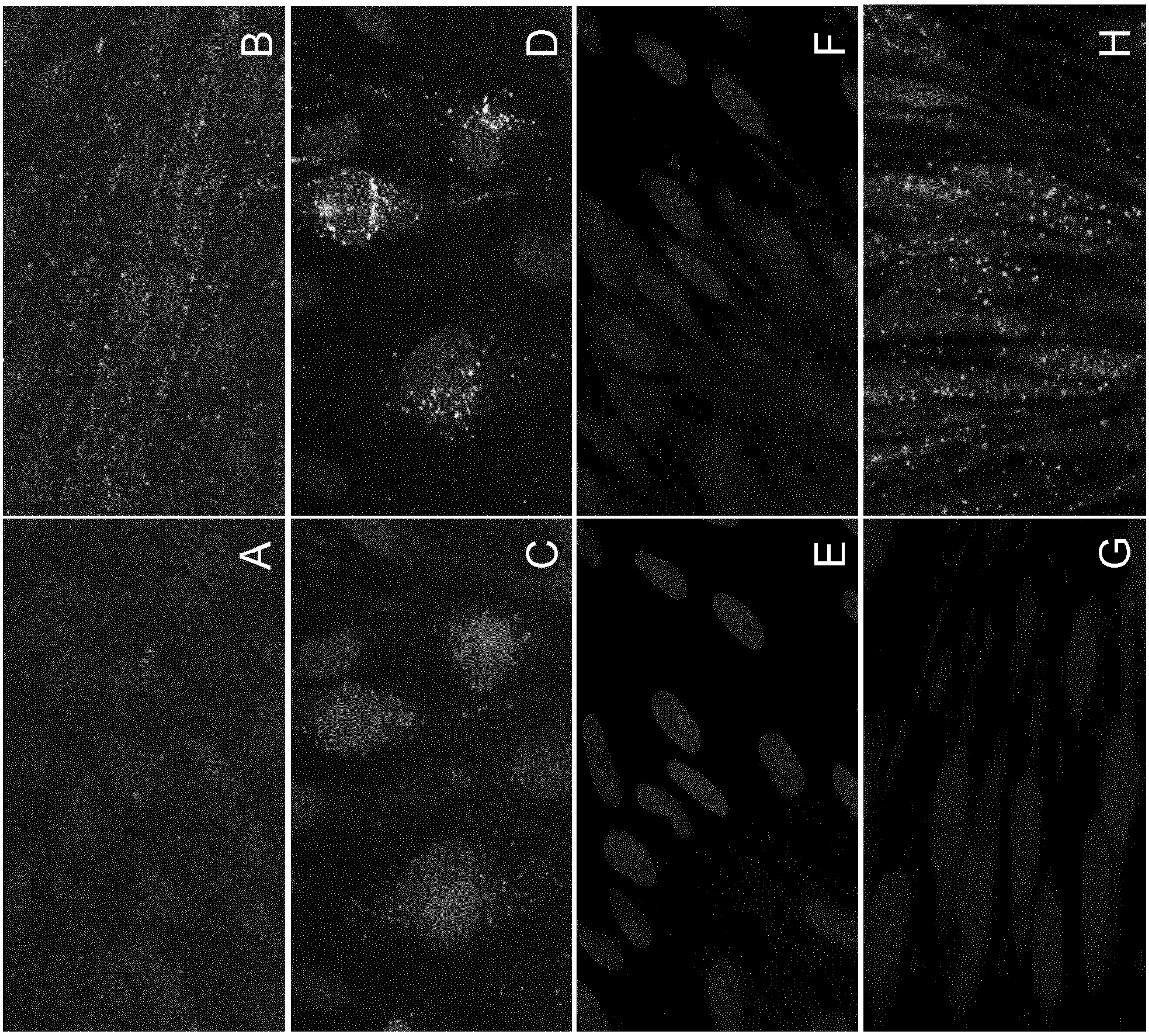

1

Figure 7

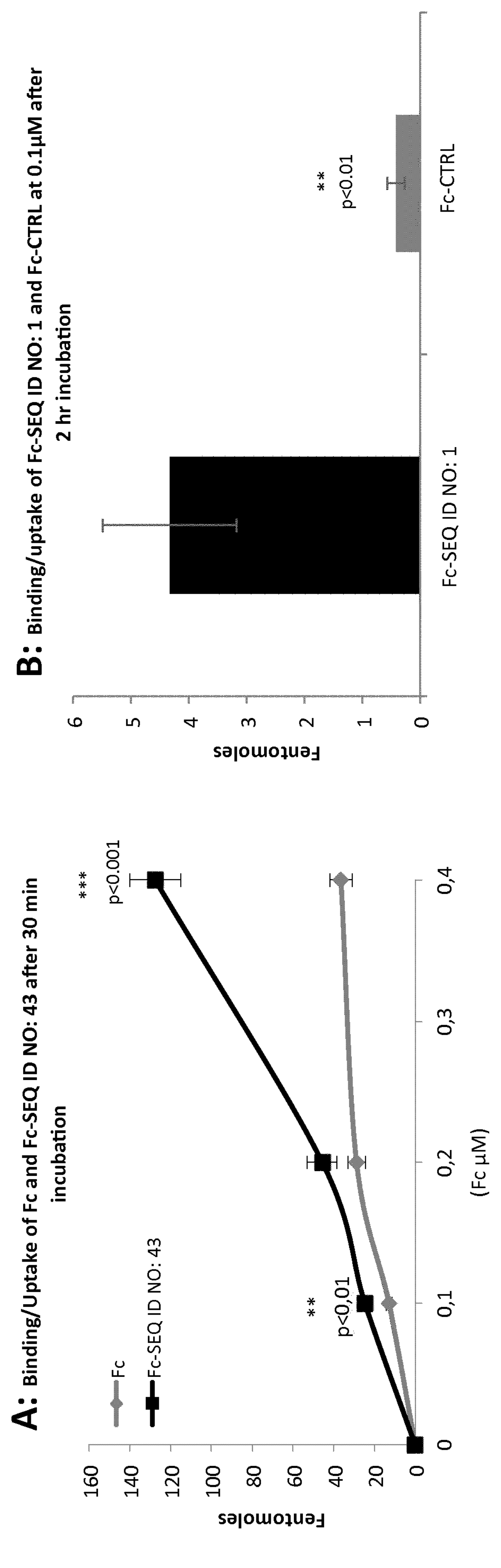
A: Binding/Uptake of Fc and Fc-SEQ ID NO: 43 after 30 min incubation
Fc
Fc-SEQ ID NO: 43
Fentomoles
160
140
120
100
80
60
40
20
0
0
0,1
0,2
0,3
0,4
(Fc µM)
**
p<0,01
***
p<0.001
B: Binding/uptake of Fc-SEQ ID NO: 1 and Fc-CTRL at 0.1µM after 2 hr incubation
Fentomoles
6
5
4
3
2
1
0
Fc-SEQ ID NO: 1
Fc-CTRL
**
p<0.01

# Figure 8

A:

1:

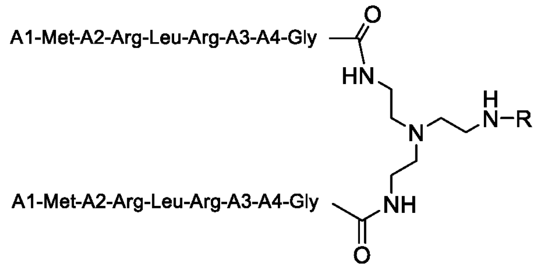

A1=(D)-Cys, A2=Pro, A3=Gl, A4=Cys, R=H: **D1(SEQ ID NO: 17)$_2$**
A1=(D)-Cys, A2=Thz, A3=Gly, A4=Pen, R=H: **D1(SEQ ID NO: 1)$_2$**

2:

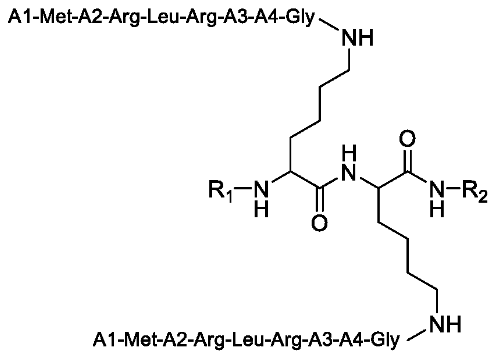

A1=(D)-Cys, A2=Thz, A3=Gly, A4=Pen, R1=H, R2=H **D2(SEQ ID NO: 1)$_2$**
A1=(D)-Cys, A2=Thz, A3=Gly, A4=Pen, R1=Cy3.5, R2=H: **D2(SEQ ID NO: 1)$_2$ -Cy3,5**

B:

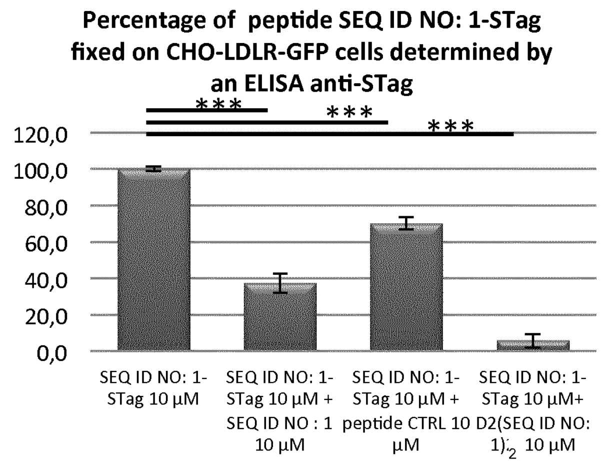


C:

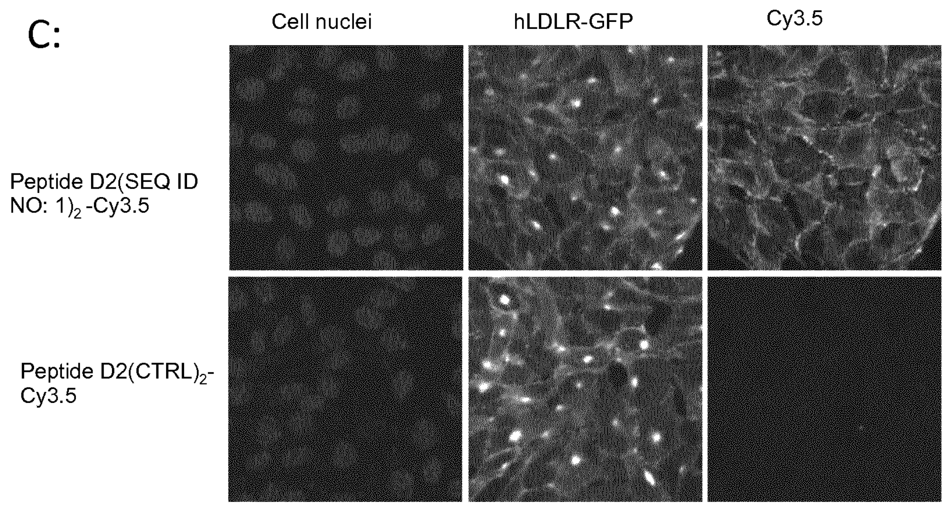

Figure 9

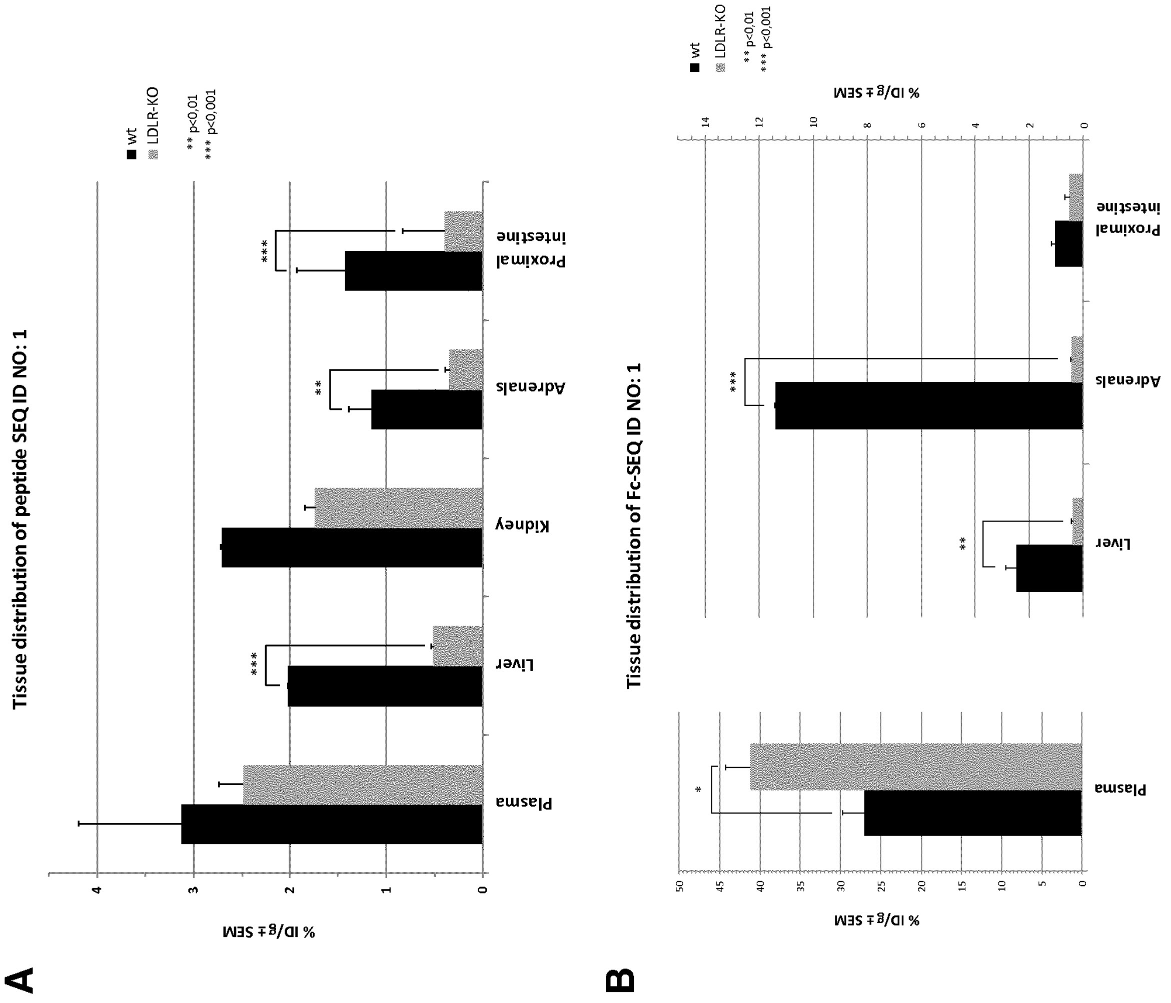
A
Tissue distribution of peptide SEQ ID NO: 1
% ID/g ± SEM
0
1
2
3
4
Plasma
Liver
Kidney
Adrenals
Proximal intestine
***
**
***
wt
LDLR-KO
** p<0,01
*** p<0,001
B
Tissue distribution of Fc-SEQ ID NO: 1
% ID/g ± SEM
0
5
10
15
20
25
30
35
40
45
50
*
Plasma
% ID/g ± SEM
0
2
4
6
8
10
12
14
Liver
Adrenals
Proximal intestine
**
***
wt
LDLR-KO
** p<0,01
*** p<0,001

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010046588 A
- WO 2011131896 A


### Non-patent literature cited in the description


- **JAIN.** Jain PharmaBiotech Report. *Drug Delivery in CNS disorders,* 2008
- **PARDRIDGE.** Pardridge. *Mol. Interv.,* 2003, vol. 3, 90-105
- **DE BOER et al.** *Clin. Pharmacokinet.,* 2007, vol. 46 (7), 553-576
- **NEUWELT et al.** *Lancet Neurol.,* 2008, vol. 7, 84-96
- **VISIONGAIN.** Ophthalmics. Visiongain, Inc, 2007
- **JANORIA KG et al.** Novel approaches to retinal drug delivery. *Expert Opin Drug Deliv.,* 2007, vol. 4 (4), 371-388
- **PARDRIDGE.** *Mol. Interv.,* 2003, vol. 3, 90-105
- **PAJOUHESH et al.** *NeuroRx,* 2005, vol. 2 (4), 541-553
- **PARDRIDGE.** *Pharm. Res.,* 2007, vol. 24 (9), 1733-1744
- **DE BOER et al.** *Annu. Rev. Pharmacol. Toxicol.,* 2007, vol. 47, 327-355
- **JONES et al.** *Pharm. Res.,* 2007, vol. 24 (9), 1759-1771
- **VLIEGHE ; KHRESTCHATISKY.** *Med Res Rev.,* 2012, vol. 33 (3), 457-516
- **AIRD.** *Exp. Neurol.,* 1984, vol. 86, 342-358
- **BABA et al.** *J. Cereb. Blood Flow Metab.,* 1991, vol. 11, 638-643
- **SALAHUDDIN et al.** *Acta Neuropathol.,* 1988, vol. 76, 1-10
- **ZHOU et al.** *J. Control. Release,* 1992, vol. 19, 459-486
- **LEVIN.** *J. Med. Chem.,* 1980, vol. 23, 682-684
- **SCHACKERT et al.** *Selective Cancer Ther.,* 1989, vol. 5, 73-79
- **KREUTER.** *Adv Drug Deliv Rev.,* 2012
- **PINZÓN-DAZA et al.** *Br J Pharmacol.,* 2012
- **JEFFERIES et al.** *Nature,* 1984, vol. 312, 162-163
- **FRIDEN et al.** *Science,* 1983, vol. 259, 373-377
- **FRIDEN.** *Neurosurgery,* 1994, vol. 35, 294-298
- **TSERENTSOODOL et al.** *Molecular Vision,* 2006, vol. 12, 1306-18
- **CHUNG ; WASAN.** *Adv Drug Deliv Rev.,* 07 May 2004, vol. 56 (9), 1315-34
- **BEISIEGEL et al.** *J Biol Chem.,* 1981, vol. 25;256 (8), 4071-8
- **HUETTINGER et al.** *J Clin Invest.,* 1984, vol. 74 (3), 1017-26
- **FONG et al.** *J Clin Invest,* 1989, vol. 84 (3), 847-56
- **VARSHOSAZ et al.** *Eur J Med Chem,* 2012, vol. 54, 429-38
- **KOPECKA et al.** *Journal of Controlled Release,* 2011, vol. 149, 196-205
- **NIKANJAM et al.** *Int J Pharm.,* 2007, vol. 328 (1), 86-94
- **NG et al.** *Acc Chem Res,* 2011, vol. 44 (10), 1105-13
- **FIRESTONE.** *Bioconjug Chem.,* 1994, vol. 5 (2), 105-13
- **ISSANDOU et al.** *Biochem Pharmacol.,* 2004, vol. 67 (12), 2281-9
- **LAMBERT et al.** *Atherosclerosis,* 2009, vol. 203 (1), 1-7
- **COX.** *J Pathol.,* 2012, vol. 226 (2), 241-54
- **LACHMANN.** *Curr Opin Pediatr.,* 2011, vol. 23 (6), 588-93
- **HENRICKSSON et al.** *Lancet,* 1989, vol. 2 (8673), 1178-1180
- **PITAS et al.** *J. Biol. Chem.,* 1987, vol. 262, 14352-14360
- **MALENTISKA et al.** *Cancer Res.,* 2000, vol. 60, 2300-2303
- **NIKANJAM et al.** *Int. J. Pharm.,* 2007, vol. 328, 86-94
- **CHEN et al.** *Int. J. Cancer,* 2001, vol. 91, 41-45
- **NIENDORF et al.** *Int. J. Cancer,* 1995, vol. 61, 461-464
- **TATIDIS et al.** *Biochem. Pharmacol.,* 2002, vol. 63, 2169-2180
- **CARUSO et al.** *Anticancer Res.,* 2001, vol. 21, 429-433
- **GRAZIANI et al.** *Gynecol. Oncol.,* 2002, vol. 85, 493-497

- **MOLINA et al.** *J. Hepatol.,* 2007, vol. 46 (3), 411-419
- **POELTRA et al.** *J Control Release,* 2012, vol. 161 (2), 188-97
- Cleavage, Deprotection, and Isolation of Peptides after Fmoc Synthesis. *Applied Biosystems,* 1998
- **YU et al.** *Sci Transl Med.,* 25 May 2011, vol. 3 (84), 84ra44
- **FOLTZ et al.** *J Nutr.,* January 2010, vol. 140 (1), 117-8
- **DEHOUCK et al.** *J. Neurochem.,* 1990, vol. 54, 1798-1801
- **TEMSAMANI et al.** *Drug Discov. Today,* 2004, vol. 23, 1012-1019
- **MOLEMA et al.** Drug targeting, organ-specific strategies. *Methods and principles in medicinal chemistry,* 2001, vol. 12
- **KRATZ et al.** Prodrug Strategies in Anticancer Chemotherapy. *ChemMedChem,* 2008, vol. 3
- **SAITO et al.** *Adv. Drug Deliv. Rev.,* 2003, vol. 55, 199-215
- **GREENWALD et al.** *Adv. Drug Deliv. Rev.,* 2003, vol. 55, 217-250
- **D'SOUZA ; TOPP.** Release from Polymeric Prodrugs: Linkages and Their Degradation. *Journal of Pharmaceutical Science,* 2001, vol. 93
- **BILDSTEIN.** Prodrug-based Intracellular Delivery of Anticancer Agents. *Advanced Drug Delivery Review,* 2011, vol. 63
- **DAGENAIS et al.** *J Cereb Blood Flow Metab.,* 2000, vol. 20 (2), 381-6
- **DEMEULE et al.** *J. Neurochem.,* 2008, vol. 106 (4), 1534-1544